(19)

Europäisches
Patentamt

European
Patent Office

Office européen
des brevets

(11)  **EP 3 387 998 A1**

(12)  **EUROPEAN PATENT APPLICATION**
published in accordance with Art. 153(4) EPC

(43) Date of publication:
**17.10.2018 Bulletin 2018/42**

(21) Application number: **16872831.9**

(22) Date of filing: **24.11.2016**

(51) Int Cl.:
**A61B 8/12** *(2006.01)*

(86) International application number:
**PCT/JP2016/084797**

(87) International publication number:
**WO 2017/098931 (15.06.2017 Gazette 2017/24)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **08.12.2015 JP 2015239433**

(71) Applicant: **Olympus Corporation
Hachioji-shi, Tokyo 192-8507 (JP)**

(72) Inventor: **KAWASHIMA, Tomonao
Hachioji-shi
Tokyo 192-8507 (JP)**

(74) Representative: **Gunzelmann, Rainer
Wuesthoff & Wuesthoff
Patentanwälte PartG mbB
Schweigerstraße 2
81541 München (DE)**

(54) **ULTRASONIC DIAGNOSTIC APPARATUS, OPERATION METHOD FOR ULTRASONIC DIAGNOSTIC APPARATUS, AND OPERATION PROGRAM FOR ULTRASONIC DIAGNOSTIC APPARATUS**

(57) An ultrasound diagnosis device according to the present invention is an ultrasound diagnosis device configured to generate an ultrasound image based on an ultrasound signal obtained by an ultrasound probe including an ultrasound transducer that transmits ultrasound to an observation target and receives ultrasound back-scattered by the observation target. The ultrasound diagnosis device includes: a feature calculating unit configured to calculate a feature based on an ultrasound signal received from the observation target; an estimating unit configured to estimate a physical quantity of scattering bodies included in the observation target, by using a relation derived based on a known physical quantity of a reference object and a feature obtained from the target object, the reference object including scattering bodies having the known physical quantity, and the feature of the observation target calculated by the feature calculating unit; and a physical quantity information generating unit configured to generate information to be displayed on a display unit, the information including a result of the estimation by the estimating unit.

FIG.1

EP 3 387 998 A1

**Description**

Field

The present invention relates to: an ultrasound diagnosis device for observation of a tissue to be observed by use of ultrasound; an operation method of the ultrasound diagnosis device; and an operation program for the ultrasound diagnosis device.

Background

Conventionally known as a technique for observation of tissue characteristics of an observation target, such as a specimen, by use of ultrasound, is a technique for imaging of features of a frequency spectrum of an ultrasound signal obtained by reception of an ultrasound echo back-scattered by the observation target via an ultrasound transducer (see, for example, Patent Literature 1). Being scattered means that the direction of the sound wave is changed by collision or interaction with a particle in a medium. Further, being back-scattered means that the scattered sound wave is returned toward the sound source. This phenomenon is also generally called reflection, but in this application, hereinafter, the term, back-scattering, is used. The sound source in this case is the ultrasound transducer. According to this technique, after the features of the frequency spectrum are extracted as analytic values representing the tissue characteristics of the observation target, feature images are generated and displayed, the feature images having been added with visual information corresponding to the features, for example, color information. An operator, such as a doctor, diagnoses the tissue characteristics of the specimen by looking at the feature images displayed.

Citation List

Patent Literature

Patent Literature 1: International Publication WO No. 2012/011414

Summary

Technical Problem

In determining tissue characteristics, grasping sizes and a number density of scattering bodies that an observation target has is important. However, in the technique described in Patent Literature 1, the features of the frequency spectrum are not associated with physical quantities such as a diameter and a number density of the scattering bodies that the observation target has, and determination of the tissue characteristics from the color information added according to the features requires skill. Accordingly, a technique allowing tissue characteristics to be easily determined without the need for skill has been demanded.

The present invention has been made in view of the above, and an object thereof is to provide: an ultrasound diagnosis device that enables easy and accurate determination of tissue characteristics based on features; an operation method of the ultrasound diagnosis device; and an operation program for the ultrasound diagnosis device. Solution to Problem

To solve the problem described above and to achieve the object, an ultrasound diagnosis device according to the present invention is an ultrasound diagnosis device configured to generate an ultrasound image based on an ultrasound signal obtained by an ultrasound probe including an ultrasound transducer that transmits ultrasound to an observation target and receives ultrasound back-scattered by the observation target. The ultrasound diagnosis device includes: a feature calculating unit configured to calculate a feature based on an ultrasound signal received from the observation target; an estimating unit configured to estimate a physical quantity of scattering bodies included in the observation target, by using: a relation derived based on a known physical quantity of a reference object and a feature obtained from the target object, the reference object including scattering bodies having the known physical quantity; and the feature of the observation target calculated by the feature calculating unit; and a physical quantity information generating unit configured to generate information to be displayed on a display unit, the information including a result of the estimation by the estimating unit.

In the ultrasound diagnosis device according to the present invention, the physical quantity includes at least one of a number density of the scattering bodies included in the reference object, a size of the scattering bodies, and a scattering intensity of the scattering bodies.

In the ultrasound diagnosis device according to the present invention, the physical quantity includes a scattering intensity of the scattering bodies included in the reference object, and the scattering intensity is at least one of an amplitude reflectance of the scattering bodies and a medium, an energy reflectance of the scattering bodies and a medium, and a function of the scattering bodies and a medium.

In the ultrasound diagnosis device according to the present invention, the estimating unit is configured to estimate the physical quantity of the scattering bodies included in the observation target by substituting the feature calculated by the feature calculating unit into a relational expression serving as the relation.

The ultrasound diagnosis device according to the present invention further includes: a relation information storage unit configured to store therein at least one of: the relational expression; a coefficient of the relational expression; a constant term of the relational expression; and a table describing therein the relation. The estimating unit is configured to estimate the physical quantity of the scattering bodies included in the observation target, by referring to the relation information storage unit.

In the ultrasound diagnosis device according to the present invention, the relational expression is derived by multiple regression analysis of at least a part of the physical quantity and the feature.

In the ultrasound diagnosis device according to the present invention, if the known physical quantity of the scattering bodies includes a number density of the scattering bodies and a size of the scattering bodies, the multiple regression analysis is executed by non-linear transformation of the number density of the scattering bodies and the scattering bodies.

In the ultrasound diagnosis device according to the present invention, the feature includes a frequency feature calculated based on the ultrasound signal.

In the ultrasound diagnosis device according to the present invention, the feature includes an attenuation factor calculated based on the ultrasound signal.

In the ultrasound diagnosis device according to the present invention, the feature includes a sound velocity calculated based on the ultrasound signal.

In the ultrasound diagnosis device according to the present invention, the physical quantity information generating unit is configured to generate image data added with visual information according to the physical quantity estimated by the estimating unit.

In the ultrasound diagnosis device according to the present invention, if the estimating unit estimates plural physical quantities different from one another, the physical quantity information generating unit is configured to generate the information displayed on the display unit concurrently, sequentially, or at different timings, for the plural physical quantities.

The ultrasound diagnosis device according to the present invention further includes a variable transformation unit configured to execute, on at least one of the number density of the scattering bodies that has been subjected to the non-linear transformation and the size of the scattering bodies that has been subjected to the non-linear transformation, further non-linear transformation.

The ultrasound diagnosis device according to the present invention further includes an attenuation correction unit configured to correct influence of attenuation in the feature calculated based on an ultrasound signal received from the observation target. The estimating unit is configured to estimate the physical quantity of the scattering bodies included in the observation target by using: a relation derived based on the known physical quantity of the reference object including the scattering bodies having the known physical quantity and a value resulting from correction of influence of attenuation in the feature obtained from the reference object; and a value of the feature of the observation target corrected by the attenuation correction unit.

An operation method of an ultrasound diagnosis device according to the present invention is an operation method of an ultrasound diagnosis device configured to generate an ultrasound image based on an ultrasound signal obtained by an ultrasound probe including an ultrasound transducer that transmits ultrasound to an observation target and receives ultrasound back-scattered by the observation target. The operation method includes: a feature calculating step of a feature calculating unit calculating a feature based on an ultrasound signal received from the observation target; an estimating step of an estimating unit estimating a physical quantity of scattering bodies included in the observation target, by using: a relation derived based on a known physical quantity of a reference object and a feature obtained from the reference object, the reference object including scattering bodies having the known physical quantity; and the feature of the observation target calculated by the feature calculating unit; and a physical quantity information generating step of a physical quantity information generating unit generating information to be displayed on a display unit, the information including a result of the estimation by the estimating unit.

An operation program for an ultrasound diagnosis device according to the present invention is an operation program for an ultrasound diagnosis device configured to generate an ultrasound image based on an ultrasound signal obtained by an ultrasound probe including an ultrasound transducer that transmits ultrasound to an observation target and receives ultrasound back-scattered by the observation target. The operation program causes the ultrasound diagnosis device to execute: feature calculating of a feature calculating unit calculating a feature based on an ultrasound signal received from the observation target; estimating of an estimating unit estimating a physical quantity of scattering bodies included in the observation target, by using: a relation derived based on a known physical quantity of a target object and a feature obtained from the reference object, the reference object including scattering bodies having the known physical quantity; and the feature of the observation target calculated by the feature calculating unit; and physical quantity information generating of a physical quantity information generating unit generating information to be displayed on a display unit, the information including a result of the estimation by the estimating unit. Advantageous Effects of Invention

The present invention has an effect of enabling easy and accurate determination of tissue characteristics based on features.

Brief Description of Drawings

FIG. 1 is a block diagram illustrating a configuration of an ultrasound diagnosis system including an ultrasound diagnosis device according to a first embodiment of the present invention.

FIG. 2 is a diagram illustrating a relation between reception depth and amplification factor in amplification processing executed by a signal amplifying unit of the ultrasound diagnosis device according to the first embodiment of the present invention.

FIG. 3 is a diagram schematically illustrating a scanning region of an ultrasound transducer and B-mode reception data.

FIG. 4 is a diagram schematically illustrating a data array in one sound ray of an ultrasound signal.

FIG. 5 is a diagram illustrating an example of spectral data calculated by a frequency analysis unit of the ultrasound diagnosis device according to the first embodiment of the present invention.

FIG. 6 is a diagram illustrating a straight line having, as parameters, corrected features that have been corrected by an attenuation correction unit of the ultrasound diagnosis device according to the first embodiment of the present invention.

FIG. 7 is a diagram illustrating a relation among mid-band fit, and diameter and number density of scattering bodies, and is a diagram illustrating a regression plane.

FIG. 8 is a flow chart illustrating an outline of processing executed by the ultrasound diagnosis device according to the first embodiment of the present invention.

FIG. 9 is a flow chart illustrating an outline of processing executed by the frequency analysis unit of the ultrasound diagnosis device according to the first embodiment of the present invention.

FIG. 10 is a diagram schematically illustrating an example of display of a feature image on a display device of the ultrasound diagnosis device according to the first embodiment of the present invention.

FIG. 11 is a block diagram illustrating a configuration of an ultrasound diagnosis system including an ultrasound diagnosis device according to a modified example of the first embodiment of the present invention.

FIG. 12 is a diagram schematically illustrating an example of display of a feature image on a display device of the ultrasound diagnosis device according to the first embodiment of the present invention.

FIG. 13 is a block diagram illustrating a configuration of an ultrasound diagnosis system including an ultrasound diagnosis device according to a third embodiment of the present invention.

FIG. 14 is a block diagram illustrating a configuration of an ultrasound diagnosis system including an ultrasound diagnosis device according to a fourth embodiment of the present invention.

FIG. 15 is a diagram for explanation of a lookup table stored in the ultrasound diagnosis device according to the fourth embodiment of the present invention.

Description of Embodiments

Hereinafter, by reference to the appended drawings, modes for implementation of the present invention (hereinafter, referred to as "embodiments") will be described.

First Embodiment

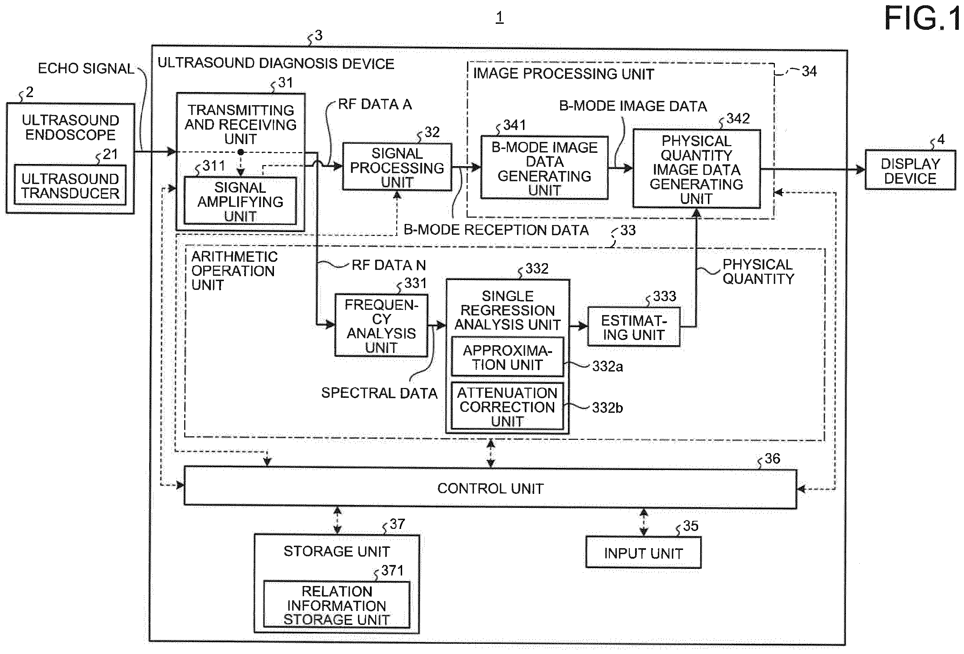

FIG. 1 is a block diagram illustrating a configuration of an ultrasound diagnosis system 1 including an ultrasound diagnosis device 3 according to a first embodiment of the present invention. The ultrasound diagnosis system 1 illustrated in FIG. 1 includes: an ultrasound endoscope 2 that transmits ultrasound to an observation target that is a target to be observed, and receives the ultrasound back-scattered by the observation target; an ultrasound diagnosis device 3 that generates, based on an ultrasound signal obtained by the ultrasound endoscope 2, an ultrasound image; and a display device 4 that displays thereon the ultrasound image generated by the ultrasound diagnosis device 3. One ultrasound endoscope 2 may be connected, or plural ultrasound endoscopes 2, regardless of whether the plural ultrasound endoscopes 2 are of the same type or different types, may be simultaneously connected, to the ultrasound diagnosis device 3. In this embodiment, the ultrasound endoscope 2 functions as an ultrasound probe. In FIG. 1, solid lined arrows represent transmission of electric signals related to an image, and broken lined arrows represent transmission of electric signals related to control.

The ultrasound endoscope 2 has, at a distal end portion thereof, an ultrasound transducer 21 that: converts an electric pulse signal received from the ultrasound diagnosis device 3, to an ultrasound pulse (an acoustic pulse); irradiates the observation target with the ultrasound pulse; and coverts an ultrasound echo back-scattered by the observation target, to an electric echo signal representing the ultrasound echo with voltage variation.

Normally, the ultrasound endoscope 2 further has, at the distal end portion thereof, an imaging optical system and an imaging element, is inserted in the gastrointestinal tract (the esophagus, stomach, duodenum, or large intestine) or the respiratory organ (the trachea or bronchus) of the observation target, and is able to capture images of the gastrointestinal tract or respiratory organ, and the organs surrounding the gastrointestinal tract or respiratory organ (the pancreas, gallbladder, bile duct, biliary tract, lymph node, mediastinal organ, blood vessel, and/or the like). The ultrasound endoscope 2 has a long insertion portion for the observation target. The insertion portion normally has a light guide that guides illumination light to be emitted to the observation target upon imaging. This light guide has a distal end portion that reaches to a distal end of the insertion portion, and a proximal end portion connected to a light source device that generates the illumination light.

The ultrasound diagnosis device 3 includes: a transmitting and receiving unit 31 that is electrically connected to the ultrasound endoscope 2, transmits, based on predetermined waveform and transmission timing, a transmission signal (a pulse signal) formed of a high voltage pulse, to the ultrasound transducer 21, receives an echo signal, which is an electric reception signal, from the ultrasound transducer 21, and generates and outputs digital data (hereinafter, referred to as RF data) of a radio frequency (RF) signal; a signal processing unit 32 that generates, based on the RF data received from the transmitting and receiving unit 31, digital B-mode reception data; an arithmetic operation unit 33 that executes predetermined arithmetic operations on the RF data received from the transmitting and receiving unit 31; an image processing unit 34 that generates various image data; an input unit 35 that is realized by use of a user interface, such as a keyboard, a mouse, and a touch panel, and receives input of various types of information; a control unit 36 that controls the whole ultrasound diagnosis system 1; and a storage unit 37 that stores therein various types of information required for operation of the ultrasound diagnosis device 3.

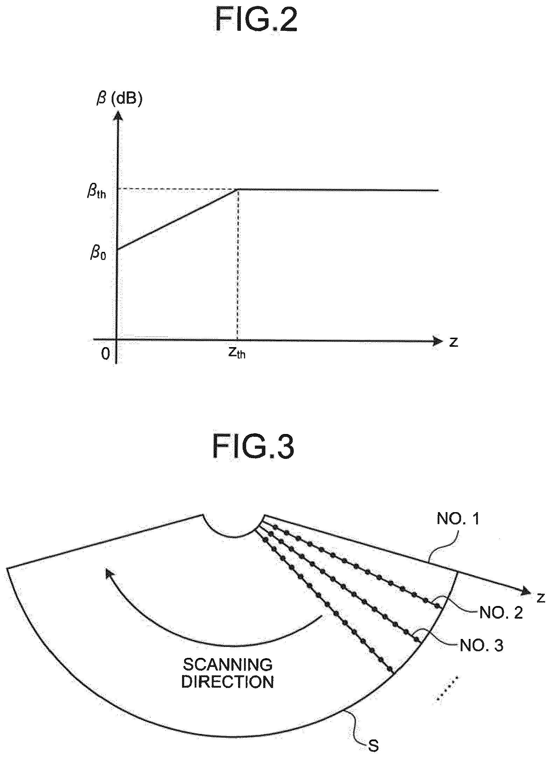

The transmitting and receiving unit 31 has a signal amplifying unit 311 that amplifies the echo signal. The signal amplifying unit 311 performs sensitivity time control (STC) correction where the larger the reception depth of the echo signal is, the larger the amplification factor, at which the echo signal is amplified, is. FIG. 2 is a diagram illustrating a relation between reception depth and amplification factor in amplification processing executed by the signal amplifying unit 311. Reception depth z illustrated in FIG. 2 is quantity calculated based on elapsed time from a time point of a start of ultrasound reception. As illustrated in FIG. 2, amplification factor $\beta$ (dB) linearly increases from $\beta_0$ to $\beta_{th}$ ($> \beta_0$) as the reception depth z increases, when the reception depth z is less than a threshold value $z_{th}$. Further, the amplification factor $\beta$ (dB) is a constant value $\beta_{th}$, when the reception depth z is equal to or greater than the threshold value $z_{th}$. The threshold value $z_{th}$ is a value, at which the ultrasound signal received from the observation target has almost attenuated and noise therein has become dominant. More generally, when the reception depth z is less than the threshold value $z_{th}$, the amplification factor $\beta$ preferably increases monotonically as the reception depth z increases. The relation illustrated in FIG. 2 is stored in the storage unit 37 beforehand.

After executing processing, such as filtering, on both the echo signal amplified by the signal amplifying unit 311, and the original echo signal that has not been amplified, the transmitting and receiving unit 31 executes sampling at an appropriate sampling frequency (for example, 50 MHz), and executes discretization (so-called A/D conversion processing). Thus, the transmitting and receiving unit 31 generates two sets of RF data, which are RF data A discretized from the non-amplified echo signal and RF data N discretized from the non-amplified echo signal, and outputs these two sets of RF data to the signal processing unit 32 and the arithmetic operation unit 33. Herein, "A" is an abbreviation for "amplified", and "N" is an abbreviation for "normal". If the ultrasound endoscope 2 has a configuration that causes the ultrasound transducer 21 to execute electronic scanning, the ultrasound transducer 21 having plural elements provided in an array, the transmitting and receiving unit 31 has a multi-channel circuit for beam combination corresponding to the plural elements.

A frequency band of the pulse signal transmitted by the transmitting and receiving unit 31 is made a wide band substantially covering a linear response frequency band for electric-acoustic conversion of the pulse signal by the ultrasound transducer 21 to an ultrasound pulse. Further, a frequency band for various types of processing on the echo signal in the signal amplifying unit 311 is made a wide band substantially covering a linear response frequency band for acoustic-electric conversion of the ultrasound echo by the ultrasound transducer 21 to an echo signal. Thereby, when later described frequency spectrum approximation processing is executed, accurate approximation is able to be executed.

The transmitting and receiving unit 31 also has functions of transmitting various control signals output by the control unit 36, to the ultrasound endoscope 2, and receiving various types of information including an ID for identification from the ultrasound endoscope 2 and transmitting the various types of information to the control unit 36.

The signal processing unit 32 executes known processing, such as bandpass filtering, envelope demodulation, and logarithmic transformation, on the RF data A, and generates digital B-mode reception data. In the logarithmic transformation, a common logarithm of a quantity resulting from division of the RF data A by a reference voltage $V_c$ is expressed as a decibel value. In these B-mode reception data, reception signal amplitudes or intensities indicating strengths of back-scattering of an ultrasound pulse are lined along a transmission and reception direction (a depth direction) of the ultrasound pulse. FIG. 3 is a diagram schematically illustrating a scanning region of the ultrasound transducer 21 (which may be, hereinafter, simply referred to as the scanning region) and the B-mode reception data. A scanning region S illustrated in FIG. 3 is fan-shaped. In FIG. 3, a path (a sound ray), on which ultrasound reciprocates, is represented by a straight line, and the B-mode reception data are represented by points lined on each sound ray. In FIG. 3, for convenience of later explanation, the sound rays are respectively numbered, "1", "2", "3", ..., in order from a start of scanning (the right in FIG. 3). The first sound ray is defined as $SR_1$, the second sound ray as $SR_2$, the third sound ray as $SR_3$, ..., and the k-th sound ray as $SR_k$. FIG. 3 corresponds to a case where the ultrasound transducer 21 is a convex transducer. Further, in FIG. 3, reception depth of the B-mode reception data is written as "z". When an ultrasound pulse emitted from a surface of the ultrasound transducer 21 is back-scattered in an object that is at a reception depth z, and is returned to the ultrasound transducer 21 as an ultrasound echo, that reciprocating distance L and the reception depth z have a relation, z = L/2. The signal processing unit 32 outputs the generated B-mode reception data to a B-mode image data generating unit 341 of the image processing unit 34. The signal processing unit 32 is realized by use of, for example: a general purpose processor, such as a central processing unit (CPU); or a dedicated integrated circuit that executes specific functions, such as an application specific integrated circuit (ASIC), or a field programmable gate array (FPGA).

The arithmetic operation unit 33 has: a frequency analysis unit 331 that calculates spectral data by executing frequency analysis through execution of fast Fourier transform (FFT) on the RF data N generated by the transmitting and receiving unit 31; a single regression analysis unit 332 (a feature calculating unit) that calculates, by using the spectral data calculated by the frequency analysis unit 331, features of the spectral data by single regression analysis; and an estimating unit 333 that estimates, by using the features calculated by the single regression analysis unit 332 and a relational expression, which has been stored in the storage unit 37 and derived from a number density of scattering bodies (hereinafter, the number density being denoted by "n") and a diameter (hereinafter, the diameter of the scattering bodies being denoted by "d") thereof, a logarithm of the number density n (log n) and a logarithm of the diameter d (log d). The arithmetic operation unit 33 is realized by use of a central processing unit (CPU) or various arithmetic operation circuits. The number density of scattering bodies means the number of scattering bodies included in a unit volume, and in this embodiment, is the number of scattering bodies per $cm^3$. Further, the diameter d is in micrometers ($\mu$m) in this embodiment. Furthermore, logarithms of the number density and the diameter of scattering bodies are common logarithms having a base of 10. Therefore, each of log n and log d represents "the number of digits of the decimal representation of n or d - 1".

The frequency analysis unit 331 generates sample data by executing sampling again at predetermined time intervals on RF data N (line data) of each sound ray generated by the transmitting and receiving unit 31. By executing FFT processing on a sample data group, the frequency analysis unit 331 calculates frequency spectra at multiple positions (data positions) on the RF data. A "frequency spectrum" referred to herein means a "frequency distribution of intensity at a certain reception depth z" obtained by FFT processing on the sample data group. Further, the "intensity" referred to herein refers to any one of, for example: a parameter, such as a voltage of the echo signal, an electric power of the echo signal, a sound pressure of the ultrasound echo, or acoustic energy of the ultrasound echo; and an amplitude or a time-integrated value of the parameter, or a combination thereof.

In this embodiment, voltage of the echo signal is adopted as the intensity, and the frequency analysis unit 331 is described as generating data (hereinafter, also referred to as spectral data) of a frequency spectrum, based on a frequency component V(f, L) of voltage amplitude. Herein, "f" is frequency. After dividing the frequency component V(f, L) of voltage amplitude by the reference voltage Vc and executing logarithmic transformation processing for representation in decibels by finding a common logarithm (log) of the divided value, the frequency analysis unit 331 multiplies the transformed value by an appropriate positive constant A, thereby generating spectral data F(f, L) given by Equation (1) below.

$$F(f, L) = A \cdot \log\{V(f, L)/V_c\} \qquad (1)$$

Herein, log denotes "common logarithm" (hereinafter, the same applies).

Hereinafter, a method of finding a frequency component V(f, L) of voltage amplitude by frequency analysis in the frequency analysis unit 331 will be described. Generally, when the observation target is a biological tissue, a frequency spectrum of an echo signal indicates different trends depending on characteristics of the biological tissue scanned with ultrasound. This is because, a frequency spectrum has correlations to a size, a number density, an acoustic impedance, and the like of scattering bodies that scatter ultrasound. "Characteristics of a biological tissue" referred to herein are, for example, a malignant tumor (cancer), a benign tumor, an endocrine tumor, a mucinous tumor, a normal tissue, a cyst, a vessel, and the like.

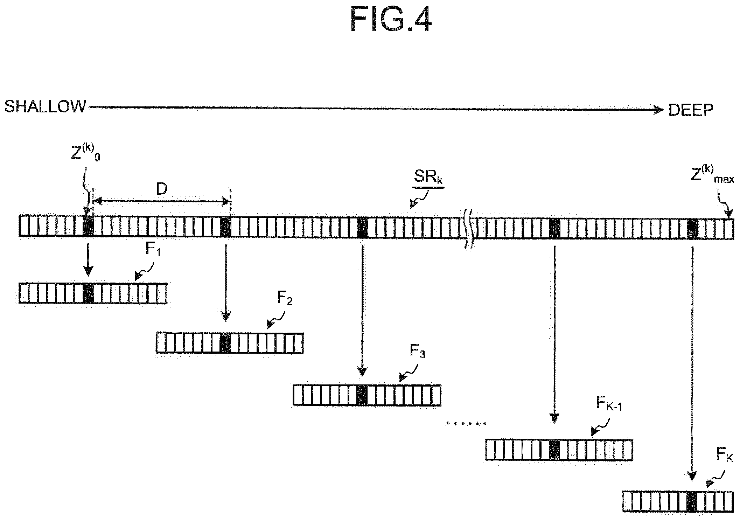

FIG. 4 is a diagram schematically illustrating a data array in one sound ray $SR_k$ of an ultrasound signal. A white or black rectangle in the sound ray $SR_k$ means data at one sample point. Further, the more rightward the data are positioned in the sound ray $SR_k$, the deeper the position where the sample data were measured along the sound ray $SR_k$ is away from the ultrasound transducer 21 (see the arrow in FIG. 4). As described already, the sound ray $SR_k$ is sample data sampled further by the frequency analysis unit 331 from RF data sampled and discretized from an echo signal by A/D conversion processing in the transmitting and receiving unit 31. FIG. 4 illustrates a case where the 8th data position of the sound ray $SR_k$ of the number k is set as an initial value $Z^{(k)}_0$ in the direction of the reception depth z, but the position of the initial value may be set arbitrarily. Results of calculation by the frequency analysis unit 331 are obtained as complex numbers and stored in the storage unit 37.

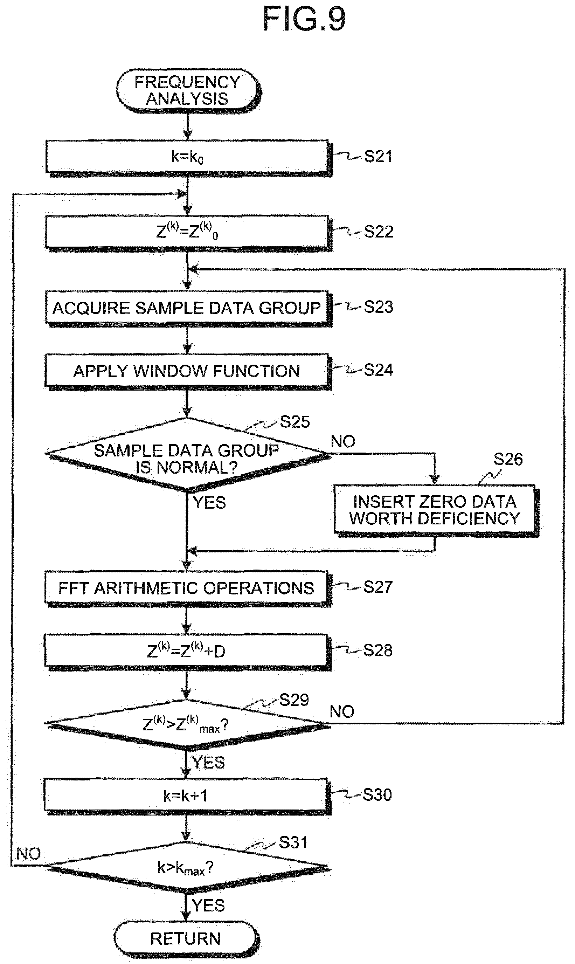

A data group $F_j$ (j = 1, 2, ..., K) illustrated in FIG. 4 is a sample data group to be subjected to FFT processing. Generally, for execution of FFT processing, the number of data that the sample data group has needs to be an exponent of 2. In that sense, the sample data group $F_j$ (j = 1, 2, ..., K-1) is a normal data group, in which the number of data is 16 (= $2^4$), while the sample data group $F_K$ is an abnormal data group since the number of data therein is 12. When FET processing is executed on an abnormal data group, processing for generation of a normal sample data group by insertion of zero data worth the deficiency is executed. This point will be described in detail when processing by the frequency analysis unit 331 is described (see FIG. 9). Thereafter, as described already, the frequency analysis unit 331 executes FET processing, calculates a frequency component V(f, L) of voltage amplitude, calculates spectral data F(f, L) based on Equation (1) mentioned above, and outputs the spectral data F(f, L) to the single regression analysis unit 332.

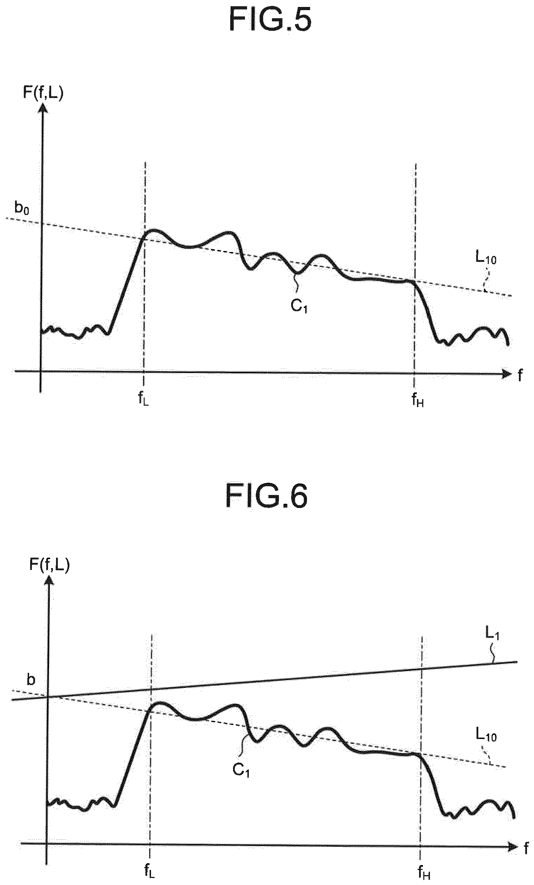

FIG. 5 is a diagram illustrating an example of spectral data calculated by the frequency analysis unit 331. In FIG. 5, the horizontal axis represents frequency f. Further, in FIG. 5, the vertical axis represents spectral data F(f, L) given by Equation (1) above. A straight line $L_{10}$ illustrated in FIG. 5 will be described later. In this embodiment, a curve and a straight line are each formed of a group of discrete points.

In spectral data $C_1$ illustrated in FIG. 5, a lower limit frequency $f_L$ and an upper limit frequency $f_H$, which are used in subsequent arithmetic operations, are parameters determined based on a frequency band of the ultrasound transducer 21, a frequency band of the pulse signal transmitted by the transmitting and receiving unit 31, and the like. Hereinafter, with respect to FIG. 5, a frequency band determined by the lower limit frequency $f_L$ and the upper limit frequency $f_H$ will be referred to as a "frequency band U".

The single regression analysis unit 332 has: an approximation unit 332a that calculates, by approximating plural spectral data output from the frequency analysis unit 331 by a straight line, features (hereinafter, referred to as uncorrected features) of the spectral data; and a attenuation correction unit 332b that calculates features by correcting attenuation dependent on frequency in the uncorrected features calculated by the approximation unit 332a.

By approximating the spectral data by a linear expression (a regression line) by execution of single regression analysis on spectral data in a predetermined frequency band, the approximation unit 332a calculates uncorrected features characterizing the approximating linear equation. Single regression analysis is regression analysis where the number of types of independent variables is only one. An independent variable of the single regression analysis in this embodiment is frequency f. For example, when spectral data are in a state of $C_1$ illustrated in FIG. 5, the approximation unit 332a obtains, by executing single regression analysis in the frequency band U, the straight line $L_{10}$ that is a regression line of the spectral data $C_1$. Next, the approximation unit 332a calculates uncorrected features, which are: a slope $a_0$ and an intercept $b_0$ of the straight line $L_{10}$; and a mid-band fit, $c_0 = a_0 f_M + b_0$, which is a value on the regression line at a center frequency of the frequency band U (that is, "a mid-band"), $f_M = (f_L + f_H)/2$. By representation of the spectral data $C_1$ by parameters (the gradient $a_0$, the intercept $b_0$, and the mid-band fit $c_0$) of the linear expression characterizing the straight line $L_{10}$ as described above, approximation of the spectral data $C_1$ by a linear expression is achieved.

Of these three uncorrected features, the slope $a_0$ and the intercept $b_0$ are considered to have correlations to the size of scattering bodies scattering ultrasound, the scattering intensity of scattering bodies, the number density (concentration) of scattering bodies, and the like. The mid-band fit $c_0$ provides the intensity of the spectrum at the center of the effective frequency band. Therefore, the mid-band fit $c_0$ is considered to have a certain degree of correlation to brightness of a B-mode image, in addition to the size of scattering bodies, the scattering intensity of scattering bodies, and the number density of scattering bodies. Thereafter, the approximation unit 332a outputs these uncorrected features $a_0$, $b_0$, and $c_0$, to the attenuation correction unit 332b. The approximation unit 332a may approximate spectral data by a polynomial expression of degree 2 or more through regression analysis.

Correction executed by the attenuation correction unit 332b will now be described. Generally, amplitude of ultrasound exponentially attenuates. Therefore, when an amplitude is logarithmically transformed into a decibel representation, an attenuation A(f, z) caused in a period, in which ultrasound reciprocates between the reception depth 0 and the reception depth z, is able to be expressed as a linear change before and after the reciprocation (a difference in decibel representation). It is known that this attenuation A(f, z) of amplitude is dependent on frequency when the observation target is a living body, and that the attenuation is large when the frequency is high and the attenuation is small when the frequency is low. In particular, the attenuation is empirically known to be proportional to frequency in a uniform tissue, and is expressed by Equation (2) below.

$$A(f, z) = 2\zeta z f \qquad\qquad (2)$$

Herein, the proportionality constant $\zeta$, is a quantity called an attenuation factor. Further, z is the reception depth of ultrasound, and f is frequency. When the observation target is a living body, a specific value of the attenuation factor $\zeta$ is determined according to the region or the tissue in the living body. In a normal liver, the value is generally 0.55 dB/cm/MHz. In this embodiment, values of the attenuation factor $\zeta$ are stored in the storage unit 37 beforehand, and the attenuation correction unit 332b reads and uses a value of the attenuation factor $\zeta$ from the storage unit 37, as appropriate. If the input unit 35 receives input of a region name or a tissue name of the observation target beforehand from an operator before transmission of ultrasound by the ultrasound endoscope 2, the attenuation correction unit 332b reads an appropriate value of the attenuation factor $\zeta$ corresponding to the region name or tissue name, and uses the value in the following attenuation correction. Further, if the input unit 35 directly receives a value of the attenuation factor $\zeta$ from the operator, the attenuation correction unit 332b uses that value in the following attenuation correction. If the input unit 35 does not receive any input from the operator, the attenuation correction unit 332b uses 0.55 dB/cm/MHz mentioned above, in the following attenuation correction.

The attenuation correction unit 332b calculates corrected features a, b, and c (hereinafter, simply referred to as the features) by executing attenuation correction according to Equations (3) to (5) below, on the uncorrected features (the slope $a_0$, the intercept $b_0$, and the mid-band fit $c_0$) extracted by the approximation unit 332a.

$$a = a_0 + 2\zeta z \qquad\qquad (3)$$

$$b = b_0 \qquad\qquad (4)$$

$$c = c_0 + A(f_M, z) = c_0 + 2\zeta z f_M ( = a f_M + b) \qquad\qquad (5)$$

As evident from Equations (3) and (5), the larger the reception depth z of ultrasound is, the larger the correction amount of the correction executed by the attenuation correction unit 332b. Further, according to Equation (4), the correction related to the intercept is identical transformation. This is because, the intercept is a frequency component corresponding to a frequency of 0 (Hz) and is not influenced by the attenuation.

FIG. 6 is a diagram illustrating a straight line having parameters, which are the features a, b, and c calculated by the attenuation correction unit 332b. Where "I" is a value on the vertical axis of FIG. 6, the equation of a straight line $L_1$ is expressed as follows.

$$I = a f + b = (a_0 + 2\zeta z) f + b_0 \qquad\qquad (6)$$

As evident from this Equation (6), the straight line $L_1$ has, as compared to the straight line $L_{10}$ before the attenuation correction, a larger slope ($a > a_0$) and the same intercept ($b = b_0$). Thereafter, the single regression analysis unit 332 outputs these features a, b, and c that have been subjected to the attenuation correction, to the estimating unit 333.

The estimating unit 333 estimates, by using the feature a and feature c, on which the single regression analysis unit 333 has executed the attenuation correction, and constants $\alpha$, $\beta$, $\gamma$, $\alpha'$, $\beta'$, and $\gamma'$ stored in the storage unit 37, a number density n and a diameter d, which are practically common logarithms, log n and log d, that are orders thereof. Specifically, assuming that the feature a (the slope) and the feature c (the mid-band fit) have a linear relation to orders (the number of digit - 1) that are approximate values of the number density n of scattering bodies and the diameter d of scattering bodies, that is, to log n and log d, the estimating unit 333 estimates log n and log d, based on Equation (10) derived from the following Equations (7) and (8) of the regression plane. The estimating unit 333 outputs the estimated log n and/or log d as physical quantities or a physical quantity, to the image processing unit 34.

Hereinafter, preparation for estimation of unknown log n and log d from the observation target by the estimating unit 333 will be described. Firstly, a reference object is prepared. In this embodiment, plural reference phantoms artificially made will be described as an example of reference objects. A reference phantom is formed by: mixture of many particles serving as scattering bodies into a uniform medium; and solidification of the mixture. The same material is used for the scattering bodies such that their mass densities, sound velocities, and acoustic impedances are made equal beforehand. Scattering bodies are sieved such that the scattering bodies having the same diameter are sorted out. Further, the scattering bodies are mixed evenly in the medium such that unevenness is not caused, and the number density of the scattering bodies in the reference phantom is also uniform. As described above, the material, the mass density, the sound velocity, the acoustic impedance, the diameter, and the number density, of scattering bodies in a reference phantom are of known values, and are uniform in the reference phantom. The material, the mass density, the sound velocity, and the acoustic impedance, of the medium, are also of known values, and are uniform in the reference phantom. Accordingly, in a reference phantom, the scattering bodies and the medium are even and uniform, and thus the attenuation factor [dB/cm/MHz] of the reference phantom is also uniform. The plural reference phantoms are generated by change of these parameters.

Next, features $c_1$, $c_2$, $c_3$, ..., and $c_N$ (mid-band fits) are obtained based on ultrasound echoes obtained by transmission of ultrasound to N reference phantoms known to have different combinations of number densities of scattering bodies and diameters of scattering bodies, $(n_1, d_1)$, $(n_2, d_2)$, $(n_3, d_3)$, ..., and $(n_N, d_N)$. In the following description, $n_i$ is the number density of scattering bodies of an i-th reference phantom of the N phantoms, $d_i$ is the diameter thereof, $\zeta_i$ is the attenuation factor thereof, and $c_i$ is the feature (the mid-band fit) obtained from this i-th reference phantom ($1 \leq i \leq N$). Influence of attenuation is eliminated from the feature $c_i$ by use of the depth, at which the spectral data of the i-th reference phantom are calculated and Equation (5), based on the attenuation factor $\zeta_i$ of the i-th reference phantom, and thus the feature $c_i$ is not dependent on the depth. As a result, N data sets $(c_1, n_1, d_1)$, $(c_2, n_2, d_2)$, $(c_3, n_3, d_3)$, ..., and $(c_N, n_N, d_N)$, each of which is formed of a number density of scattering bodies, a diameter of the scattering bodies, and a feature, are obtained.

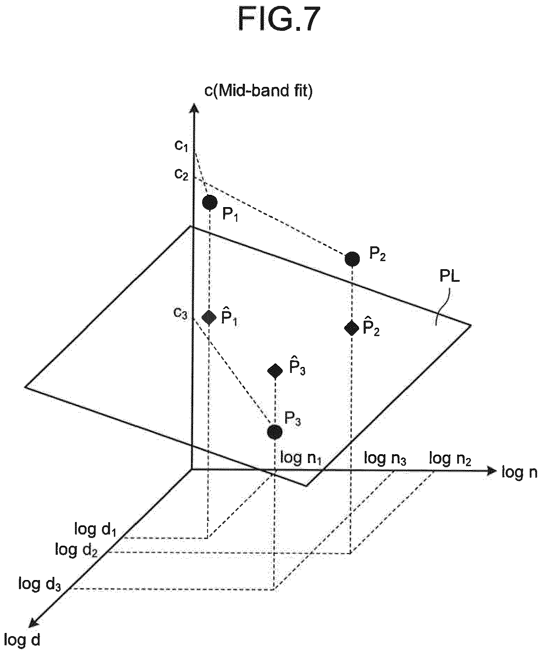

Next, a relation among the number density of scattering bodies, the diameter of scattering bodies, and the feature is found from the N data sets. FIG. 7 illustrates an orthogonal coordinate system having the mid-band fit (feature c), the common logarithm (log n) of the number density of scattering bodies, and the common logarithm (log d) of the diameter of scattering bodies, plotted along axes orthogonal to one another. In this first embodiment, a regression plane PL of a plot $P_1(\log n_1, \log d_1, c_1)$ corresponding to the first reference phantom, a plot $P_2(\log n_2, \log d_2, c_2)$ corresponding to the second reference phantom, a plot $P_3(\log n_3, \log d_3, c_3)$ corresponding to the third reference phantom, ..., and a plot $P_N(\log n_N, \log d_N, c_N)$ corresponding to the N-th reference phantom is found beforehand by use of the least squares method. The regression plane PL of the plots $P_1$, $P_2$, $P_3$, ..., and $P_N$ with respect to the feature c is a plane where the sum of squares of differences (errors) from the plots $P_1$, $P_2$, $P_3$, ... , and $P_N$, that is, distances of line segments $P_1P_1{}^\wedge$, $P_2P_2{}^\wedge$, $P_3P_3{}^\wedge$, ..., and $P_NP_N{}^\wedge$, is minimized. Herein, $P_i{}^\wedge$ is a point of intersection between a straight line passing the plot $P_i$ and parallel to an axis of interest (herein, the coordinate axis for the feature c) and this plane. Finding the regression plane PL by use of the least squares method as described above and performing analysis based on the regression plane PL corresponds to multiple regression analysis. Multiple regression analysis is regression analysis where the number of types of independent variables is two or more. Independent variables of the multiple regression analysis in this embodiment are the diameter d of scattering bodies and the number density n of scattering bodies.

Hereinafter, a method of specifically finding the regression plane PL will be described. Generally, an equation of a plane is able to be written as a linear equation. Since the regression plane PL with respect to the feature c illustrated in FIG. 7 is in a three-dimensional space, the feature c is able to be written as an equation (Equation (7)) representing the feature c by a linear combination of the two variables (log n, log d). Herein, $\alpha$, $\beta$, and $\gamma$ are constants of real numbers determining the plane. Finding the regression plane PL with respect to the feature c is equivalent to finding the values of $\alpha$, $\beta$, and $\gamma$. A regression plane PL for the feature a is also able to be considered, similarly to the feature c, and is able to be written as an equation (Equation (8)) representing the feature a by a linear combination of the two variables (log n, log d). Herein, $\alpha'$, $\beta'$, and $\gamma'$ are constants of real numbers determining the plane. Finding the regression plane PL with respect to the feature a is equivalent to finding the values of $\alpha'$, $\beta'$, and $\gamma'$.

$$c = \alpha \cdot \log n + \beta \cdot \log d + \gamma \qquad (7)$$

$$a = \alpha' \cdot \log n + \beta' \cdot \log d + \gamma' \qquad (8)$$

From Equations (7) and (8) above, Equation (9) below is obtained.

$$\begin{pmatrix} c \\ a \end{pmatrix} = \begin{pmatrix} \alpha & \beta \\ \alpha' & \beta' \end{pmatrix} \begin{pmatrix} \log n \\ \log d \end{pmatrix} + \begin{pmatrix} \gamma \\ \gamma' \end{pmatrix} \qquad (9)$$

From Equation (9) above, Equation (10) below is obtained.

$$\begin{pmatrix} \log n \\ \log d \end{pmatrix} = \begin{pmatrix} \alpha & \beta \\ \alpha' & \beta' \end{pmatrix}^{-1} \left\{ \begin{pmatrix} c \\ a \end{pmatrix} - \begin{pmatrix} \gamma \\ \gamma' \end{pmatrix} \right\} \qquad (10)$$

How the constants $\alpha$, $\beta$, $\gamma$, $\alpha'$, $\beta'$, and $\gamma'$ are found will now be described. Hereinafter, as an example, how the constants $\alpha$, $\beta$, and $\gamma$ are found will be described. After the feature $c_i$ is obtained from each of the N reference phantoms, averages over all of the reference phantoms are found respectively for the number density $n_i$, the diameter $d_i$, and the feature $c_i$ of scattering bodies. Thereafter, a matrix G and a column vector Y represented by Equations (11) and (12) below are defined. The coefficients $\alpha$ and $\beta$ of the equation of the regression plane are able to be obtained by Equation (13) below defined by Equation (11) below and Equation (12) below.

$$G = \begin{pmatrix} \log n_1 - \log \bar{n} & \log n_2 - \log \bar{n} & \cdots & \log n_{N-1} - \log \bar{n} & \log n_N - \log \bar{n} \\ \log d_1 - \log \bar{d} & \log d_2 - \log \bar{d} & \cdots & \log d_{N-1} - \log \bar{d} & \log d_N - \log \bar{d} \end{pmatrix} \qquad (11)$$

$$Y = \begin{pmatrix} c_1 - \bar{c} \\ c_2 - \bar{c} \\ c_3 - \bar{c} \\ \vdots \\ c_N - \bar{c} \end{pmatrix} \qquad (12)$$

$$\begin{pmatrix} \alpha \\ \beta \end{pmatrix} = \left( G^t G \right)^{-1} G Y \qquad (13)$$

The left superscript "t" therein denotes "transposed matrix" and the right superscript "-1" denotes "inverse matrix".
Further, since the regression plane PL passes a point (the centroid of $P_1$, $P_2$, ... , and $P_N$) having coordinates at the averages for the number density $n_i$, the diameter $d_i$, and the feature $c_i$ of scattering bodies, from Equation (7), Equation (14) below is obtained. By substitution of $\alpha$ and $\beta$ obtained by Equation (13) above into Equation (14) below, the constant $\gamma$ is able to be found.

$$\bar{c} = \alpha \cdot \bar{n} + \beta \cdot \bar{d} + \gamma \qquad (14)$$

As described above, by use of the data sets $(c_1, n_1, d_1)$, $(c_2, n_2, d_2)$, $(c_3, n_3, d_3)$, ... , and $(c_N, n_N, d_N)$ related to the N reference phantoms, the constants $\alpha$, $\beta$, and $\gamma$ of Equation (7) above are able to be found. As to the constants $\alpha'$, $\beta'$, and $\gamma'$ of Equation (8) above, similarly, by use of data sets $(a_1, n_1, d_1)$, $(a_2, n_2, d_2)$, $(a_3, n_3, d_3)$, ... , and $(a_N, n_N, d_N)$ related to the N reference phantoms, the constants $\alpha'$, $\beta'$, and $\gamma'$ are able to be found.
Hereinbefore, the preparation for estimation of unknown log n and log d from the observation target by the estimating unit 333 through determination of the constants $\alpha$, $\beta$, $\gamma$, $\alpha'$, $\beta'$, and $\gamma'$ has been described. The feature c and feature a of the observation target obtained via the ultrasound endoscope 2, as well as log d that is "the number of digits of the diameter d of scattering bodies in the observation target - 1", and log n that is "the number of digits of the number density n of these scattering bodies - 1" are also considered to follow the physical trends obtained from the reference phantoms. Therefore, when a data set (n, d, c) from the observation target is plotted in FIG. 7, the values are expected to be estimated to be somewhere on the regression plane PL. Therefore, this data set (n, d, c) satisfies Equation (7) of the regression plane PL. Similarly, a data set (n, d, a) from the observation target also satisfies Equation (8). Since n, d, c, and a of the observation target satisfy Equation (7) and Equation (8), they satisfy Equation (10). Therefore, the estimating unit 333 estimates log n and log d by substituting the feature c and feature a from the observation target into Equation (10) as described above.

In this embodiment, the above described reception of the echo signals based on the ultrasound echoes from the reference phantoms, calculation of the feature c and feature a, calculation of the N data sets, and calculation of the constants $\alpha$, $\beta$, $\gamma$, $\alpha'$, $\beta'$, and $\gamma'$ are executed outside the ultrasound diagnosis device 3. The obtained constants $\alpha$, $\beta$, $\gamma$, $\alpha'$, $\beta'$, and $\gamma'$ are stored in a relation information storage unit 371 built in the storage unit 37, via the input unit 35, before factory shipment. An important point in this embodiment is that the estimating unit 333 estimates log n and log d based on the multiple regression analysis method by using features based on echo signals from reference objects with known physical quantities.

With reference back to FIG. 1, the image processing unit 34 has: a B-mode image data generating unit 341 that generates B-mode image data representing amplitude of an echo signal by brightness; and a physical quantity image data generating unit 342 (a physical quantity information generating unit) that generates physical quantity image data for display of the B-mode image data generated by the B-mode image data generating unit 341 and the physical quantities (log n and/or log d) estimated by the estimating unit 333, in association with visual information, together with a B-mode image.

The B-mode image data generating unit 341 generates the B-mode image data by executing signal processing using known techniques such as gain processing and contrast processing, on the B-mode reception data received from the signal processing unit 32, and executing thinning or the like of data according to a data step width determined according to an image display range in the display device 4. The B-mode image is a gray scale image having red (R), green (G), and blue (B) values matching one another, the R, G, and B values being variables when the RGB color system is adopted as the color space.

After executing coordinate transformation, by which the B-mode reception data from the signal processing unit 32 are rearranged so as to enable correct spatial representation of a scanning range, the B-mode image data generating unit 341 fills in gaps among the B-mode reception data by executing interpolation processing among the B-mode reception data, and generates B-mode image data. The B-mode image data generating unit 341 outputs the generated B-mode image data to the physical quantity image data generating unit 342.

The physical quantity image data generating unit 342 generates the physical quantity image data by superimposing the visual information related to the physical quantity estimated by the estimating unit 333 on each pixel of the image of the B-mode image data. For a pixel area corresponding to a data amount of one sample data group $F_j$ (j = 1, 2, ..., K) illustrated in FIG. 4, for example, the physical quantity image data generating unit 342 assigns visual information related to a physical quantity corresponding to a feature of a frequency spectrum calculated from the sample data group $F_j$. The physical quantity image data generating unit 342 generates physical quantity image data by associating any one of log n and log d with hue serving as visual information. The physical quantity image data generating unit 342 may generate the physical quantity image data by associating one of log n and/or log d with hue, and associating the other with contrast. Examples of the visual information related to the physical quantities include variables of a color space forming a predetermined color system, such as, for example, hue, chroma, brightness, luminance, red (R), green (G), and blue (B).

Generated as the physical quantity image data generated by the physical quantity image data generating unit 342 are physical quantity image data, by which a physical quantity image of an area according to a region of interest (ROI) defined by specific depth width, sound ray width, and the like in the scanning region S illustrated in FIG. 3 is displayed on the display device 4.

The control unit 36 is realized by use of: a general-purpose processor, such as a CPU, which has arithmetic operation and controlling functions; a dedicated integrated circuit, such as an ASIC or an FPGA; or the like. The control unit 36 integrally controls the ultrasound diagnosis device 3 by reading information recorded and stored in the storage unit 37 from the storage unit 37 and executing various types of arithmetic operation processing related to an operation method of the ultrasound diagnosis device 3. The control unit 36 may be configured by use of the general purpose processor, the dedicated integrated circuit, or the like, which is common to the signal processing unit 32 and arithmetic operation unit 33.

The storage unit 37 stores therein plural features calculated by the attenuation correction unit 332b for each frequency spectrum, and image data generated by the image processing unit 34. Further, the storage unit 37 has the relation information storage unit 371 that stores therein the relational expressions (the constants $\alpha$, $\beta$, $\gamma$, $\alpha'$, $\beta'$, and $\gamma'$) for the estimation processing executed by the estimating unit 333.

The storage unit 37 stores therein, in addition to the above, for example, information needed in the amplification processing (the relation between amplification factor and reception depth illustrated in FIG. 2), information needed in the logarithmic transformation processing (as seen in Equation (1), for example, the values of A and $V_c$), information on a window function needed in the frequency analysis processing (Hamming, Hanning, Blackman, or the like), and the like.

Further, the storage unit 37 stores therein various programs including an operation program for execution of the operation method of the ultrasound diagnosis device 3. The operation program may be recorded in a computer readable recording medium, such as a hard disk, a flash memory, a CD-ROM, a DVD-ROM, or a flexible disk, and widely distributed. The above mentioned various programs may be obtained by being downloaded via a communication network. The communication network referred to herein is realized by, for example, an existing public network, a local area network (LAN), a wide area network (WAN), or the like, and may be wired or wireless.

The storage unit 37 having the above configuration is realized by use of: a read only memory (ROM) having the various programs installed therein beforehand; and a random access memory (RAM), a hard disk, or the like, storing therein arithmetic operation parameters, data, and the like for processing.

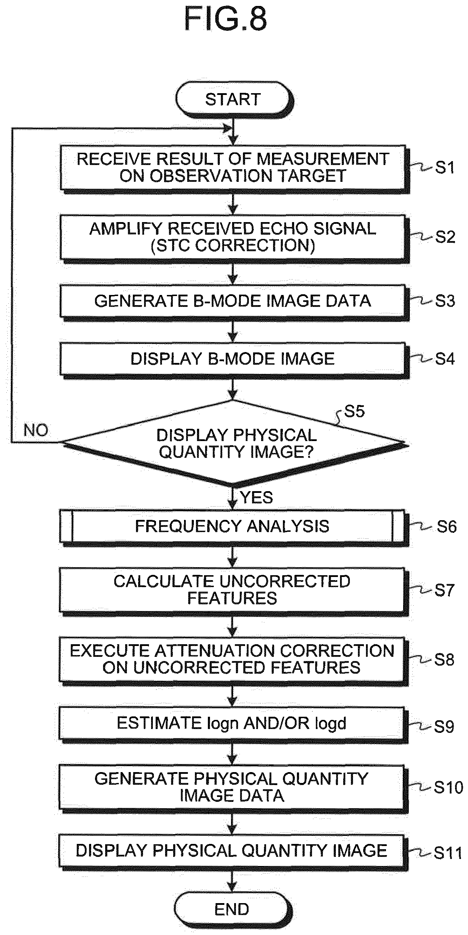

FIG. 8 is a flow chart illustrating an outline of processing executed by the ultrasound diagnosis device 3 having the above configuration. Firstly, the ultrasound diagnosis device 3 receives an echo signal that is a result of measurement on an observation target by the ultrasound transducer 21, from the ultrasound endoscope 2 (Step S1).

The signal amplifying unit 311 that has received the echo signal from the ultrasound transducer 21 executes amplification of that echo signal (Step S2). The signal amplifying unit 311 executes, based on the relation between the amplification factor and the reception depth illustrated in FIG. 2, for example, amplification (STC correction) of the echo signal. Next, the transmitting and receiving unit 31 samples, at an appropriate sampling frequency (for example, 50 MHz) both the amplified echo signal and the original echo signal that has not been amplified, discretizes the sampled echo signals, generates RF data A and RF data N respectively therefrom, and outputs the former to the B-mode image data generating unit 341, and the latter to the frequency analysis unit 331.

Subsequently, the B-mode image data generating unit 341 generates B-mode image data by using the RF data A output from the transmitting and receiving unit 31, and outputs the B-mode image data to the physical quantity image data generating unit 342 (Step S3). The physical quantity image data generating unit 342 outputs the B-mode image data to the display device 4 as is, without processing the B-mode image data. The display device 4 that has received the B-mode image data displays thereon a B-mode image corresponding to the B-mode image data (Step S4) .

Thereafter, the control unit 36 checks which of "display" and "non-display" of a physical quantity image has been selected by a user, such as an operator, via a button or menu of the input unit 35, the button or menu not being illustrated in the drawings (Step S5). The control unit 36 outputs a physical quantity image generation start command to each unit forming the arithmetic operation unit 33 if the control unit 36 confirms that "display" has been selected (Step S5: Yes). If it is confirmed that "non-display" has been selected, the physical quantity image generation start command is not output (Step S5: No). When the units of the arithmetic operation unit 33 receive the physical quantity image generation start command, the units execute later described processing of Step S6 and after. Regardless of the presence or absence of the physical quantity image generation start command, the transmitting and receiving unit 31, the signal amplifying unit 311, the signal processing unit 32, the B-mode image data generating unit 341, and the physical quantity image data generating unit 342, of the ultrasound diagnosis device 3 repeat the above described processing from Step S1 to Step S4. Therefore, while the user is specifying, through the input unit 35, "non-display" of the physical quantity image, the B-mode image is repeatedly displayed on the display device 4 every time scanning in the observation target by the ultrasound transducer 21 is executed.

If the units of the arithmetic operation unit 33 receive the physical quantity image generation start command, firstly, the frequency analysis unit 331 calculates spectral data for all of sample data groups by executing frequency analysis through FFT processing on the RF data N (Step S6: frequency analysis step). FIG. 9 is a flow chart illustrating an outline of processing executed by the frequency analysis unit 331 at Step S6. Hereinafter, by reference to the flow chart illustrated in FIG. 9, the frequency analysis processing will be described in detail.

Firstly, the frequency analysis unit 331 sets the counter k for identification of a sound ray to be analyzed, to $k_0$ (Step S21). This initial value $k_0$ is the number for the rightmost sound ray in the analysis range in FIG. 3.

Subsequently, the frequency analysis unit 331 sets the initial value $Z^{(k)}_0$ of data position (corresponding to reception depth) $Z^{(k)}$ representing a group of a series of data (a sample data group) obtained for FFT processing (Step S22). For example, as described above, FIG. 4 illustrates the case where the 8th data position on the sound ray $SR_k$ is set as the initial value $Z^{(k)}_0$. This initial value $Z^{(k)}_0$ is the shallowest reception depth in the analysis range on the sound ray $SR_k$.

Thereafter, the frequency analysis unit 331 obtains a sample data group (Step S23), and applies a window function stored in the storage unit 37 to the obtained sample data group (Step S24). By this application of the window function to the sample data group, the sample data group is able to be prevented from becoming discontinuous at a boundary, and generation of artifacts is able to be prevented.

Subsequently, the frequency analysis unit 331 determines whether or not the sample data group at the data position $Z^{(k)}$ is a normal data group (Step S25). As described when reference was made to FIG. 4, the number of data that the sample data group has needs to be equal to an exponent of 2. Hereinafter, the number of data in a normal sample data group is assumed to be $2^n$ (where n is a positive integer). In this embodiment, the data position $Z^{(k)}$ is set to be as close as possible to the center of the sample data group that $Z^{(k)}$ belongs to. Specifically, since the number of data in the sample data group is $2^n$, $Z^{(k)}$ is set at a $2^n/2$ (= $2^{n-1}$)-th position that is close to the center of that sample data group. In this case, the sample data group being normal means that there are $2^{n-1} - 1$ (= N) data on a shallower side of the data position $Z^{(k)}$ and there are $2^{n-1}$ (= M) data on a deeper side of the data position $Z^{(k)}$. In the case illustrated in FIG. 4, the sample data groups $F_1$, $F_2$, $F_3$, ..., and $F_{K-1}$ are all normal. In FIG. 4, the case where n = 4 (N = 7, M = 8) is illustrated as an example.

As a result of the determination at step S25, if the sample data group at the data position $Z^{(k)}$ is normal (step S25: Yes), the frequency analysis unit 331 proceeds to later described step S27.

As a result of the determination at step S25, if the sample data group at the data position $Z^{(k)}$ is not normal (step S25: No), the frequency analysis unit 331 generates a normal sample data group by inserting zero data worth the deficiency therein (Step S26). A window function has been applied to the sample data group determined to be not normal at Step S25 (for example, the sample data group $F_K$ in FIG. 4) before the zero data are added thereto. Therefore, even if the zero data are inserted in the sample data group, discontinuity is not caused in the data. After step S26, the frequency analysis unit 331 proceeds to later described step S27.

At Step S27, the frequency analysis unit 331 calculates V(f, L), which is a frequency component of amplitude, by executing FFT arithmetic operations by using the sample data group. Thereafter, the frequency analysis unit 331 executes logarithmic transformation processing on V(f, L), and obtains spectral data F(f, L) (Step S27).

Subsequently, the frequency analysis unit 331 changes the data position $Z^{(k)}$ by a step width D (Step S28). It is assumed that the storage unit 37 stores therein an input value for the step width D by the operator via the input unit 35 beforehand. In FIG. 4, the case where D = 15 is illustrated as an example. The step width D is desirably made as small as possible, particularly to be made to match the data step width used when the B-mode image data generating unit 341 generates the B-mode image data, but if the amount of arithmetic operations in the frequency analysis unit 331 is desired to be reduced, a value larger than the data step width may be set as the step width D.

Thereafter, the frequency analysis unit 331 determines whether or not the data position $Z^{(k)}$ is larger than the maximum value $Z^{(k)}_{max}$ in the sound ray $SR_k$ (Step S29). This maximum value $Z^{(k)}_{max}$ is the deepest reception depth in the analysis range on the sound ray $SR_k$. If the data position $Z^{(k)}$ is larger than the maximum value $Z^{(k)}_{max}$ (Step S29: Yes), the frequency analysis unit 331 increments the counter k by 1 (Step S30). This means the processing is shifted to the next sound ray. On the contrary, if the data position $Z^{(k)}$ is equal to or less than the maximum value $Z^{(k)}_{max}$ (Step S29: No), the frequency analysis unit 331 returns to Step S23.

After Step S30, the frequency analysis unit 331 determines whether or not the counter k is larger than the maximum value $k_{max}$ (Step S31). If the counter k is larger than $k_{max}$ (Step S31: Yes), the frequency analysis unit 331 ends the flow of frequency analysis processing. On the contrary, if the counter k is equal to or less than the $k_{max}$ (Step S31: No), the frequency analysis unit 331 returns to Step S22. This maximum value $k_{max}$ is the number for the leftmost sound ray in the analysis range.

As described above, the frequency analysis unit 331 executes a plural number of times of FFT arithmetic operations by depth for each of ($k_{max} - k_0 + 1$) sound rays in the analyzed target region. Results of the FFT arithmetic operations are stored, together with the reception depths and the reception directions, in the storage unit 37.

Default values including the whole scanning range in FIG. 3 for these four types of values $k_0$, $k_{max}$, $Z^{(k)}_0$, and $Z^{(k)}_{max}$ are stored beforehand in the storage unit 37, and the frequency analysis unit 331 executes the processing in FIG. 9 by reading these values as appropriate. When the default values are read, the frequency analysis unit 331 executes frequency analysis processing for the whole scanning range. However, these four types of values $k_0$, $k_{max}$, $Z^{(k)}_0$, and $Z^{(k)}_{max}$ may be changed by input of a region of interest specified by the user, such as the operator, through the input unit 35. When the values are changed, the frequency analysis unit 331 executes frequency analysis processing on only the region of interest specified and input.

Subsequently to the above described frequency analysis processing of Step S6, the single regression analysis unit 332 calculates uncorrected features of each of plural sets of spectral data obtained by the frequency analysis unit 331, and calculates features of the set of spectral data by executing attenuation correction for elimination of influence of attenuation of ultrasound, on the uncorrected features of the set of spectral data (Steps S7 to S8).

At Step S7, by executing single regression analysis on each of plural sets of spectral data according to positions in the analysis range generated by the frequency analysis unit 331, the approximation unit 332a calculates uncorrected features corresponding to the set of spectral data (Step S7). Specifically, the approximation unit 332a approximates each set of spectral data by a linear expression by executing single regression analysis, and calculates the uncorrected features, which are the slope $a_0$, the intercept $b_0$, and the mid-band fit $c_0$. For example, the straight line $L_{10}$ illustrated in FIG. 5 is a regression line obtained by approximation through single regression analysis on the spectral data $C_1$ of the frequency band U by the approximation unit 332a.

Subsequently, the attenuation correction unit 332b calculates features that have been subjected to attenuation correction by executing attenuation correction by using the attenuation factor $\zeta$, on the uncorrected features obtained by the approximation by the approximation unit 332a on each set of spectral data, and stores the calculated features in the storage unit 37 (Step S8). The straight line $L_1$ illustrated in FIG. 6 is an example of a straight line obtained by execution of attenuation correction processing by the attenuation correction unit 332b.

At Step S8, the attenuation correction unit 332b executes calculation by substituting a data position $Z = v_S/(2 \cdot f_{sp}) \cdot D \cdot n_S + Z_0$ obtained by use of the data array of the sound ray of the ultrasound signal, for the reception depth z in Equations (3) and (5) described above. Herein, $f_{sp}$ is the sampling frequency of data, $v_s$ is the sound velocity, D is the data step width, $n_S$ is the number of data steps from the first data on the sound ray up to the data position of the sample data group to be processed, and $Z_0$ is the shallowest reception depth in the analysis range. For example, if the sampling frequency $f_{sp}$ of data is 50 MHz, the sound velocity $v_s$ is 1530 m/sec, and the data step width D is 15 by adoption of the data array illustrated in FIG. 5, $z = 0.2295 n_S + Z_0$ (mm) holds.

Thereafter, the estimating unit 333 estimates log n and/or log d that are logarithms of the number density n and the diameter d, by using: the feature a and the feature c that have been subjected to the attenuation correction by the single regression analysis unit 332; and the constants $\alpha$, $\beta$, $\gamma$, $\alpha'$, $\beta'$, and $\gamma'$ stored in the relation information storage unit 371 (Step S9: estimation step). The estimating unit 333 outputs the estimated log n and/or log d, as physical quantities, to the physical quantity image data generating unit 342.

The physical quantity image data generating unit 342 generates physical quantity image data by superimposing visual information (for example, hue) associated with the physical quantity estimated at Step S9 on each pixel in the B-mode image data generated by the B-mode image data generating unit 341 (Step S10: physical quantity information generating step).

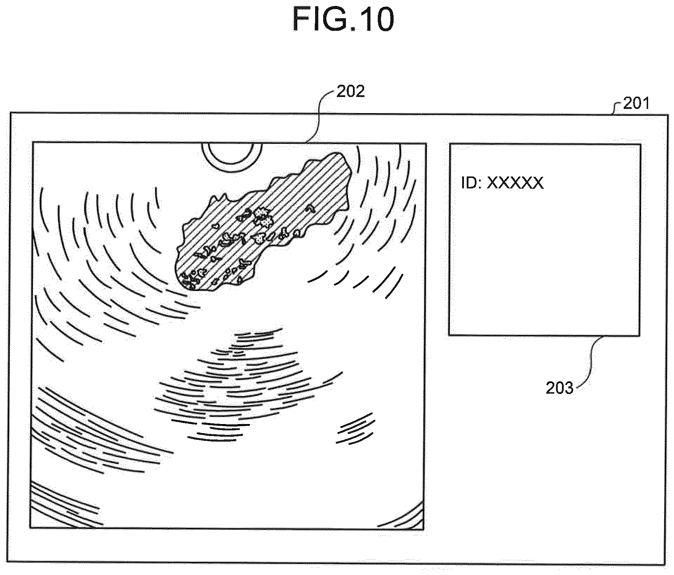

Thereafter, the display device 4 displays, under control by the control unit 36, a physical quantity image corresponding to the physical quantity image data generated by the physical quantity image data generating unit 342 (Step S11). FIG. 10 is a diagram schematically illustrating an example of display of a feature image on the display device 4. A feature image 201 illustrated in FIG. 10 has a superimposed image display section 202 that displays thereon an image having visual information superimposed on a B-mode image, the visual information being related to a physical quantity, and an information display section 203 that displays thereon identification information of the observation target. Information on the physical quantity or feature, information on an approximation equation, information on gain and contrast, or the like may be displayed further on the information display section 203. Further, a B-mode image corresponding to the physical quantity image may be displayed beside the physical quantity image.

In the above described flow of processing (Steps S1 to S11), the processing of Steps S2 to S4 and the processing of Steps S5 to S10 may be executed concurrently.

In the above described flow of processing, the example where the physical quantities of the observation target are the diameter of scattering bodies and the number density of scattering bodies, and the variable transformation is logarithmic transformation has been described. However, the embodiment is not limited to this combination, and other physical quantities may be used, and other variable transformation may be adopted. As long as the truly desired physical quantity is able to be converted to a variable, to which a feature linearly changes or gradually changes relatively, estimation is able to be executed by reduction or approximation of the transformed variable to linear multiple regression analysis, and the truly desired physical quantity is able to be estimated to a certain degree. According to this first embodiment, the order (the number of digits - 1) of each of the diameter of scattering bodies of an observation target and the number density of the scattering bodies is able to be estimated.

According to the above described first embodiment of the present invention, since the estimating unit 333 estimates log n and/or log d that are logarithms of the number density n and the diameter d by using the features calculated by the single regression analysis unit 332 and the relational expressions (the constants $\alpha$, $\beta$, $\gamma$, $\alpha'$, $\beta'$, and $\gamma'$) calculated by use of the reference phantoms, direct estimation of the number density and/or size (diameter) of scattering bodies is enabled. Thereby, without the need for skill, pathological interpretation is able to be performed easily and infallibly, the pathological interpretation being determination of a pathology corresponding to tissue characteristics corresponding to values calculated as features.

Further, according to the first embodiment of the present invention, since the constants $\alpha$, $\beta$, $\gamma$, $\alpha'$, $\beta'$, and $\gamma'$ are found based on the features that are from the reference phantoms and that have been subjected to attenuation correction, the estimation is able to be performed more accurately, independently of the differences between the attenuation factors of the observation target and the reference phantoms.

By the frequency analysis unit 331 changing $k_0$ and $k_{max}$ determining the sound ray width, and $Z^{(k)}_0$ and $Z^{(k)}_{max}$ determining the depth width, according to input of a region of interest specified by a user, such as an operator, through the input unit 35; spectral data are able to be calculated for only the region of interest defined by the specific depth width and sound ray width specified and input. Therefore, the amount of arithmetic operations related to the calculation is able to be decreased, and the frame rate is able to be increased. In this embodiment, the region of interest is defined into a fan shape by the depth width and sound ray width, but not being limited to this example, the region of interest may be rectangular, elliptical, or of any other shape. In this case, the single regression analysis unit 332 may set optimum attenuation factors individually for the set region of interest and the region outside the region of interest.

Modified Example of First Embodiment

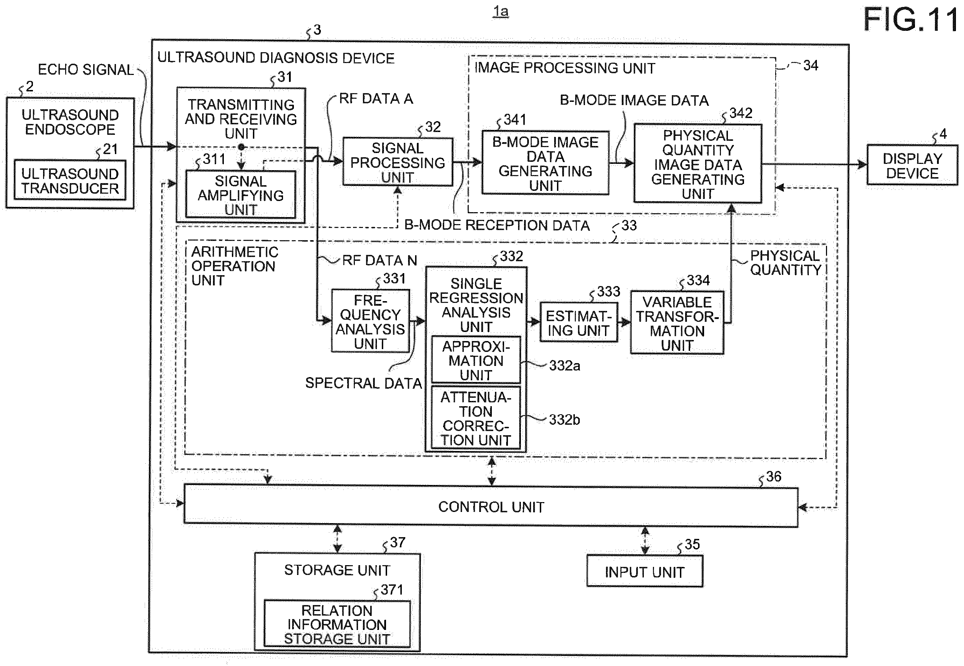

Next, a modified example of the first embodiment of the present invention will be described. According to the above description of the first embodiment, the estimating unit 333 estimates log n and/or log d that are logarithms of the number density n and the diameter d, and the physical quantity image data generating unit 342 superimposes the visual information corresponding to log n and/or log d on the B-mode image, but in this modified example, the arithmetic operation unit 33 transforms log n and/or log d estimated by the estimating unit 333 further to n and/or d. FIG. 11 is a block diagram illustrating a configuration of an ultrasound diagnosis system 1a including an ultrasound diagnosis device according to the modified example of the first embodiment of the present invention.

In the ultrasound diagnosis system 1a according to this modified example, in contrast to the above described configuration of the ultrasound diagnosis system 1 according to the first embodiment, the arithmetic operation unit 33 of the ultrasound diagnosis device 3 further includes a variable transformation unit 334. The variable transformation unit 334 transforms log n and/or log d estimated by the estimating unit 333, to n and/or d. Specifically, the estimated values for log n and/or log d are substituted for exponents of 10, for obtainment of n and/or d. The variable transformation unit 334 outputs the transformed number density n and/or diameter d to the physical quantity image data generating unit 342.

The physical quantity image data generating unit 342 generates physical quantity image data by superimposing visual information related to the physical quantities/quantity (the number density n and/or the diameter d) transformed by the variable transformation unit 334, on each pixel of the image of the B-mode image data. The physical quantity image data generating unit 342 assigns, to a pixel area corresponding to a data amount of one sample data group $F_j$ (j = 1, 2, ..., K) illustrated in FIG. 4, for example, visual information related to a physical quantity corresponding to a feature of a frequency spectrum calculated from that sample data group $F_j$.

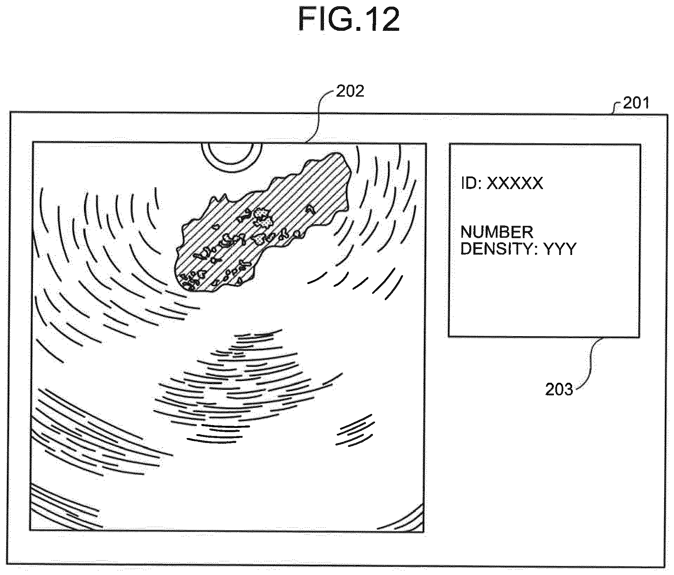

The display device 4 displays, under control by the control unit 36, a physical quantity image corresponding to the physical quantity image data generated by the physical quantity image data generating unit 342. FIG. 12 is a diagram schematically illustrating an example of display of a feature image on the display device 4. A feature image 201 illustrated in FIG. 12 has a superimposed image display section 202 that displays an image having visual information superimposed on a B-mode image, the visual information being related to a physical quantity, and an information display section 203 that displays thereon identification information of the observation target, and the like. In this modified example, the number density n and/or the diameter d of scattering bodies transformed by the variable transformation unit 334 are displayed as physical quantity information.

According to this modified example, since the number density n and the diameter d of scattering bodies are displayed as physical quantities by variable transformation of log n and/or log d estimated by the estimating unit 333, estimation by use of visual information directly related to the number density and/or size (diameter) of scattering bodies is enabled. Thereby, without the need for skill, pathological interpretation is able to be performed easily and infallibly, the pathological interpretation being determination of a pathology corresponding to tissue characteristics corresponding to values calculated as features.

Further, according to this modified example, even if the number densities n of scattering bodies in the observation target and reference phantoms and the diameters d of the scattering bodies are not directly in linear relations to the features c or the features a, log n and log a that are approximately in linear relations to the features c or features a are able to be used. By variable transformation of log n and log a, for the number density n of scattering bodies and the diameter d of scattering bodies, which are the truly desired physical quantities, not only their orders (the number of digits - 1), but also their values themselves are able to be estimated through reduction to linear multiple regression analysis.

Second Embodiment

Next, a second embodiment of the present invention will be described. The second embodiment includes a configuration that is the same as the above described configuration of the ultrasound diagnosis system including the ultrasound diagnosis device according to the first embodiment. FIG. 1 is common thereto. According to the above description of the first embodiment, the single regression analysis unit 332 calculates the features a, b, and c by single regression analysis, but in this embodiment, the single regression analysis unit 332 calculates the attenuation factor $\zeta$ in addition to these three features, as features of an observation target. The attenuation factor $\zeta$ may be calculated by use of, for example, the above described spectral data F(f, L). Specifically, the attenuation factor $\zeta$ is found by: a feature a being found by regression analysis, the feature a being a slope of a regression line for frequency f of spectral data F(f, L); a further slope of that found slope with respect to a reciprocating distance L being found next; and the further slope being multiplied by -1 further. Further, according to the above description of the first embodiment, the estimating unit 333 estimates log n and/or log d that are logarithms of the number density n and the diameter d, but in this second embodiment, the estimating unit 333 estimates a scattering intensity (hereinafter, the scattering intensity being denoted by "r"), in addition to log n and/or log d that are logarithms of the number density n and the diameter d. The scattering intensity referred to herein is the amplitude reflectance of the scattering bodies and the medium and/or the energy reflectance of the scattering bodies and the medium, and/or a function of the scattering bodies and the medium. The scattering intensity is the amplitude reflectance or energy reflectance, which is defined as follows. Herein, Z denotes acoustic impedance.

$$\text{Amplitude reflectance} = |Z_{\text{scattering body}} - Z_{\text{medium}}| / |Z_{\text{scattering body}} + Z_{\text{medium}}|$$

$$\text{Energy reflectance} = |Z_{\text{scattering body}} - Z_{\text{medium}}|^2 / |Z_{\text{scattering body}} + Z_{\text{medium}}|^2$$

In this second embodiment, the estimating unit 333 estimates log n and log d, which are logarithms of the number density n and the diameter d, as well as the scattering intensity r, by using: the feature and feature c that have been subjected to the attenuation correction by the single regression analysis unit 332 and the attenuation factor $\zeta$ serving as a feature; and the constants $\alpha$, $\beta$, $\gamma$, $\delta$, $\alpha'$, $\beta'$, $\gamma'$, $\delta'$, $\alpha''$, $\beta''$, $\gamma''$, and $\delta''$, which are stored in the storage unit 37. Specifically, on the assumption that the feature a (the slope), the feature c (the mid-band fit), and the attenuation factor $\zeta$ have linear relations to log n, log d, and the scattering intensity r; the estimating unit 333 estimates log n, log d, and the scattering intensity r, based on Equation (19) below derived from Equations (15) to (17) of a regression plane (that is, a three-dimensional object) in a four-dimensional space described later. The estimating unit 333 outputs physical quantities, which are the estimated log n, log d, and/or scattering intensity r.

$$c = \alpha \cdot \log n + \beta \cdot \log d + \gamma \cdot r + \delta \tag{15}$$

$$a = \alpha' \cdot \log n + \beta' \cdot \log d + \gamma' \cdot r + \delta' \tag{16}$$

$$\zeta = \alpha'' \cdot \log n + \beta'' \cdot \log d + \gamma'' \cdot r + \delta'' \tag{17}$$

From Equations (15) to (17) above, Equation (18) below is obtained.

$$\begin{pmatrix} c \\ a \\ \zeta \end{pmatrix} = \begin{pmatrix} \alpha & \beta & \gamma \\ \alpha' & \beta' & \gamma' \\ \alpha'' & \beta'' & \gamma'' \end{pmatrix} \begin{pmatrix} \log n \\ \log d \\ r \end{pmatrix} + \begin{pmatrix} \delta \\ \delta' \\ \delta'' \end{pmatrix} \tag{18}$$

From Equation (18) above, Equation (19) below is obtained.

$$\begin{pmatrix} \log n \\ \log d \\ r \end{pmatrix} = \begin{pmatrix} \alpha & \beta & \gamma \\ \alpha' & \beta' & \gamma' \\ \alpha'' & \beta'' & \gamma'' \end{pmatrix}^{-1} \left\{ \begin{pmatrix} c \\ a \\ \zeta \end{pmatrix} - \begin{pmatrix} \delta \\ \delta' \\ \delta'' \end{pmatrix} \right\} \tag{19}$$

How the constants $\alpha$, $\beta$, $\gamma$, $\delta$, $\alpha'$, $\beta'$, $\gamma'$, $\delta'$, $\alpha''$, $\beta''$, $\gamma''$, and $\delta''$ are found will now be described. Hereinafter, how the constants $\alpha$, $\beta$, $\gamma$, and $\delta$ are found will be described as an example. Each of these constants $\alpha$, $\beta$, $\gamma$, and $\delta$ is able to be obtained by: transmission of ultrasound to a reference phantom; and calculation of a feature c (a mid-band fit) calculated based on an ultrasound echo obtained, the reference phantom being formed by even mixture, distribution, and solidification of a material having scattering bodies therein adjusted in their size (diameter), number density, and scattering intensity, the size (diameter) and the number density of the scattering bodies being known beforehand, the attenuation factor [dB/cm/MHz] of the reference phantom being known beforehand to be uniform. In this second embodiment, similarly to the above described first embodiment, by use of N reference phantoms having different combinations of scattering body diameters, number densities, and scattering intensities, the features c are respectively calculated. In the following description, $n_i$ is the number density of scattering bodies of an i-th reference phantom of the N phantoms, $d_i$ is the diameter thereof, $r_i$ is the scattering intensity thereof, $\zeta i$ is the attenuation factor thereof, and $c_i$ is the feature (the mid-band fit) obtained from this i-th reference phantom ($1 \leq i \leq N$). Influence of attenuation is eliminated from the feature $c_i$ by use of the depth, at which the spectral data of the i-th reference phantom are calculated and Equation (5), based on the attenuation factor $\zeta_i$ of the i-th reference phantom, and thus the feature $c_i$ is not dependent on the depth.

After the feature $c_i$ is obtained from each of the N reference phantoms, averages over all of the reference phantoms are respectively found for the number density $n_i$, the diameter $d_i$, the scattering intensity $r_i$, and the feature $c_i$ of scattering bodies. Thereafter, a matrix G and a column vector Y represented by Equations (20) and (21) below are defined. The constants $\alpha$, $\beta$, and $\gamma$ of the equation of the regression plane in the four-dimensional plane are able to be obtained by Equation (22) below defined by Equations (20) and (21).

$$G = \begin{pmatrix} \log n_1 - \log \bar{n} & \log n_2 - \log \bar{n} & \cdots & \log n_{N-1} - \log \bar{n} & \log n_N - \log \bar{n} \\ \log d_1 - \log \bar{d} & \log d_2 - \log \bar{d} & \cdots & \log d_{N-1} - \log \bar{d} & \log d_N - \log \bar{d} \\ r_1 - \bar{r} & r_2 - \bar{r} & \cdots & r_{N-1} - \bar{r} & r_N - \bar{r} \end{pmatrix} \quad (20)$$

$$Y = \begin{pmatrix} c_1 - \bar{c} \\ c_2 - \bar{c} \\ c_3 - \bar{c} \\ \vdots \\ c_N - \bar{c} \end{pmatrix} \quad (21)$$

$$\begin{pmatrix} \alpha \\ \beta \\ \gamma \end{pmatrix} = \left( G^t G \right)^{-1} G Y \quad (22)$$

The left superscript "t" therein denotes "transposed matrix" and the right superscript "-1" denotes "inverse matrix".

Further, since the regression plane passes a point (the centroid) having coordinates at the averages for the number density $n_i$, the diameter $d_i$, the scattering intensity $r_i$, and the feature $c_i$ of scattering bodies, from Equation (15), Equation (23) below is obtained. By substitution of the constants $\alpha$, $\beta$, and $\gamma$ obtained from Equation (22) above into Equation (23) below, the constant $\delta$ is able to be found.

$$\bar{c} = \alpha \cdot \log \bar{n} + \beta \cdot \log \bar{d} + \gamma \cdot \bar{r} + \delta \quad (23)$$

As described above, by use of the N reference phantoms, the constants $\alpha$, $\beta$, $\gamma$, and $\delta$ of Equation (15) above are able to be found. As to the constants $\alpha'$, $\beta'$, $\gamma'$, and $\delta'$ of Equation (16) above, similarly, the constants $\alpha'$, $\beta'$, $\gamma'$, and $\delta'$ are able to be found by use of the N reference phantoms and calculation of the feature $a_i$. Further, as to the constants $\alpha''$, $\beta''$, $\gamma''$, and $\delta''$ of Equation (17) above, similarly, the constants $\alpha''$, $\beta''$, $\gamma''$, and $\delta''$ are able to be found by use of the N reference phantoms, and use of an attenuation factor $\zeta_i$ measured anew or a known attenuation factor $\zeta_i$. The obtained constants $\alpha$, $\beta$, $\gamma$, $\delta$, $\alpha'$, $\beta'$, $\gamma'$, $\delta'$, $\alpha''$, $\beta''$, $\gamma''$, and $\delta''$ are stored in the storage unit 37.

Thereafter, the feature c, the feature a, and the attenuation factor $\zeta$, of the observation target, which have been obtained through the ultrasound endoscope 2, as well as log d that is "the number of digits of the diameter d of scattering bodies in the observation target - 1", log n that is "the number of digits of the number density n of the scattering bodies - 1", and the scattering intensity r of scattering bodies are also considered to follow the physical trends obtained from the reference phantoms. Therefore, this data set (n, d, r, c) satisfies Equation (15) of the regression plane. Similarly, the data set (n, d, r, a) from the observation target satisfies Equation (16), and the data set (n, d, r, $\zeta$) from the observation target satisfies Equation (17). Since n, d, r, c, a, and $\zeta$ of the observation target satisfy Equation (15), Equations (16), and Equation (17); they satisfy Equation (19). Accordingly, the estimating unit 333 estimates log n, log d, and the scattering intensity r by substituting the feature c and feature a from the observation target and the attenuation factor $\zeta$

from the observation target serving as a feature, into Equation (19), as described above.

In this second embodiment, the above described reception of the echo signals based on the ultrasound echoes from the reference phantoms, calculation of the feature c, the feature a, and the attenuation factor $\zeta$, calculation of the N data sets, and calculation of the constants $\alpha$, $\beta$, $\gamma$, $\delta$, $\alpha'$, $\beta'$, $\gamma'$, $\delta'$, $\alpha''$, $\beta''$, $\gamma''$, and $\delta''$ are executed outside the ultrasound diagnosis device 3. The obtained constants $\alpha$, $\beta$, $\gamma$, $\delta$, $\alpha'$, $\beta'$, $\gamma'$, $\delta'$, $\alpha''$, $\beta''$, $\gamma''$, and $\delta''$ are stored in the relation information storage unit 371 built in the storage unit 37, via the input unit 35, before factory shipment.

The physical quantity image data generating unit 342 generates physical quantity image data by superimposing visual information related to the physical quantities (log n, log d, and/or r) transformed by the estimating unit 333, on each pixel of an image of the B-mode image data. The physical quantity image data generating unit 342 assigns, to a pixel area corresponding to a data amount of one sample data group $F_j$ (j = 1, 2, ..., K) illustrated in FIG. 4, for example, visual information related to a physical quantity corresponding to a feature of a frequency spectrum calculated from that sample data group $F_j$.

According to the above described second embodiment of the present invention, since the estimating unit 333 estimates log n and/or log d that are logarithms of the number density n and the diameter d, and/or the scattering intensity r, by using the features calculated by the single regression analysis unit 332, the attenuation factor, and the relational expressions (the constants $\alpha$, $\beta$, $\gamma$, $\delta$, $\alpha'$, $\beta'$, $\gamma'$, $\delta'$, $\alpha''$, $\beta''$, $\gamma''$, and $\delta''$) calculated by use of the reference phantoms; the number density, the size (the diameter), and/or the scattering density of scattering bodies are able to be directly estimated. Thereby, without the need for skill, pathological interpretation is able to be performed easily and infallibly, the pathological interpretation being determination of a pathology corresponding to tissue characteristics corresponding to values calculated as features.

Further, according to the second embodiment of the present invention, since the constants $\alpha$, $\beta$, $\gamma$, $\delta$, $\alpha'$, $\beta'$, $\gamma'$, $\delta'$, $\alpha''$, $\beta''$, $\gamma''$, and $\delta''$ are found based on the features that are from the reference phantoms and that have been subjected to attenuation correction; independently of the differences between the attenuation factors of the observation target and the reference phantoms, the log n that is "the number of digits of the number density n of scattering bodies - 1", log d that is "the number of digits of the diameter d of scattering bodies - 1", and/or the scattering intensity r are able to be estimated more accurately.

According to the above description of the second embodiment, the attenuation factor $\zeta$ is used as a physical quantity, but not being limited thereto, the variance of the feature a or c, the average frequency weighted by the spectral data, or the sound velocity may be used as a physical quantity. The sound velocity is estimated from a delay time of a received voltage for each element in a case where: the ultrasound transducer 21 having plural elements provided in an array is configured to be caused to perform electronic scanning; the transmitting and receiving unit 31 has a multi-channel circuit for beam combination corresponding to the plural elements; and conditions of focus of the echo signal from the ultrasound transducer 21 are the best. Further, the average frequency is given by Equation (24) below ($1 \leq q \leq N_f$: $N_f$ being an integer larger than 1).

$$Average\ Frequency = \left(1/N_f\right) \cdot \Sigma_q f_q \cdot F\left(f_q, L\right) \qquad (24)$$

Herein, $\Sigma_q f_q \cdot F(f_q, L)$ represents weighted addition of spectral intensities.

In the above described flow of processing, the example, where physical quantities of the observation target are the diameter of scattering bodies and the number density of scattering bodies, in addition to the scattering intensity of scattering bodies, and variable transformation is logarithmic transformation, has been described. However, not being limited to this combination, other physical quantities may be used, and other variable transformation may be adopted. As long as the truly desired physical quantity is able to be transformed to a variable, to which a feature linearly changes or gradually changes relatively; estimation is able to be executed by reduction or approximation of the transformed variable to linear multiple regression analysis and the truly desired physical quantity is able to be estimated to a certain degree. According to this second embodiment, the order (the number of digits - 1) of each of the diameter of scattering bodies and the number density of scattering bodies, of the observation target, is able to be estimated.

Third Embodiment

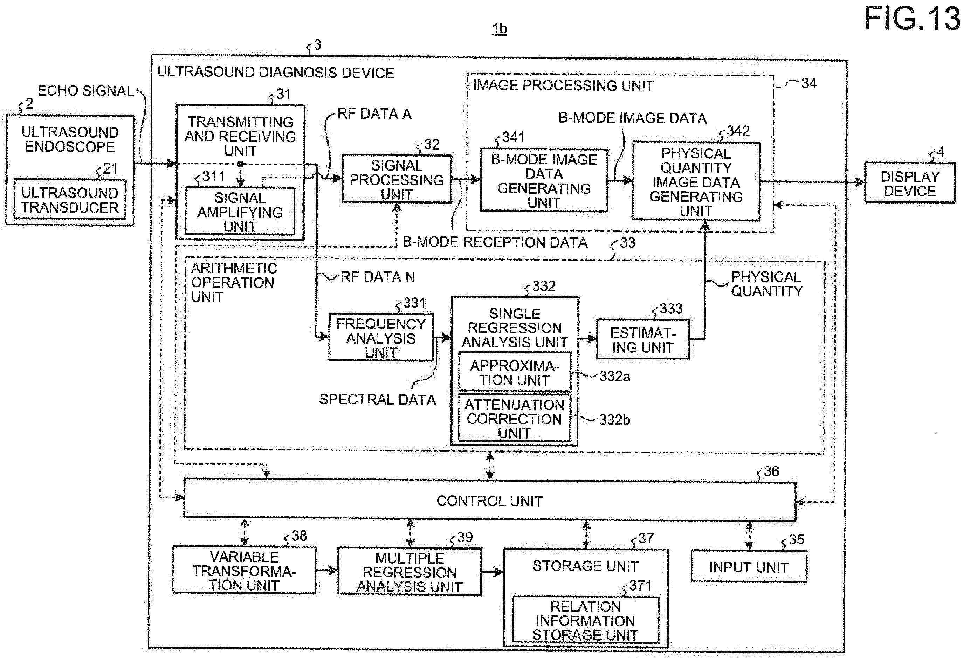

Next, a third embodiment of the present invention will be described. According to the above description of the first embodiment, the constants $\alpha$, $\beta$, $\gamma$, $\alpha'$, $\beta'$, and $\gamma'$ based on ultrasound echoes from the reference phantoms are calculated, not in the ultrasound diagnosis device, but outside the ultrasound diagnosis device, and are stored beforehand in the relation information storage unit 371; but in this third embodiment, an ultrasound diagnosis system 1b is configured to be able to calculate the constants $\alpha$, $\beta$, $\gamma$, $\alpha'$, $\beta'$, and $\gamma'$. FIG. 13 is a block diagram illustrating a configuration of an ultrasound diagnosis system including an ultrasound diagnosis device according to the third embodiment of the present invention.

In the ultrasound diagnosis system 1b according to this third embodiment, in contrast to the above described configuration of the ultrasound diagnosis system 1 according to the first embodiment, the ultrasound diagnosis device 3 further includes a variable transformation unit 38 and a multiple regression analysis unit 39.

Further, the variable transformation unit 38 obtains the number density $n_i$ and diameter $d_i$ of scattering bodies of the N reference phantoms and the attenuation factor $\zeta_i$, via the input unit 35 or the storage unit 37 ($1 \leq i \leq N$). The variable transformation unit 38 obtains $\log n_i$ and $\log d_i$ by transforming the obtained number density $n_i$ and diameter $d_i$ to logarithms. The variable transformation unit 38 outputs $\log n_i$, $\log d_i$, and the attenuation factor $\zeta_i$, to the storage unit 37. This processing is executed for all of the N reference phantoms.

Next, the ultrasound transducer 21 transmits ultrasound to an i-th reference phantom, of the N reference phantoms. Thereafter, the transmitting and receiving unit 31, the frequency analysis unit 331, and the single regression analysis unit 332 execute processing similar to the processing executed on the echo signal from the observation target in the first embodiment, on an echo signal from the i-th reference phantom. The ultrasound diagnosis system 1b thus calculates a feature $c_i$, and a feature $a_i$, based on an ultrasound echo from the i-th reference phantom ($1 \leq i \leq N$). In the calculation, the attenuation correction unit 332b eliminates influence of attenuation from the feature $c_i$ and the feature $a_i$ beforehand, by: obtaining the attenuation factor $\zeta_i$ of the i-th reference phantom from the storage unit 37; using the depth, at which the spectral data of the i-th reference phantom have been calculated, Equation (3), and Equation (5); and correcting the attenuation. Since the attenuation is uniform in the reference phantom, these feature $c_i$ and feature $a_i$ are not dependent on the depth. The single regression analysis unit 332 then outputs the feature $c_i$ and feature $a_i$ of scattering bodies of the i-th reference phantom, to the storage unit 37. The ultrasound diagnosis system 1b executes this processing on all of the N reference phantoms.

The multiple regression analysis unit 39 obtains $\log n_i$, $\log d_i$, the feature $c_i$, and the feature $a_i$ for all of the N reference phantoms from the storage unit ($1 \leq i \leq N$). The multiple regression analysis unit 39 then finds averages over all of the N reference phantoms respectively for the number density $n_i$, diameter $d_i$, feature $c_i$, and feature $a_i$ of scattering bodies. Thereafter, the multiple regression analysis unit 39 finds the constants $\alpha$, $\beta$, $\gamma$, $\alpha'$, $\beta'$, and $\gamma'$ of Equations (7) and (8) above by using Equations (11) to (14) above by the same arithmetic operation method as the first embodiment. The multiple regression analysis unit 39 outputs the found constants $\alpha$, $\beta$, $\gamma$, $\alpha'$, $\beta'$, and y' or relational expressions to the relation information storage unit 371. The relation information storage unit 371 stores therein the received constants $\alpha$, $\beta$, $\gamma$, $\alpha'$, $\beta'$, and $\gamma'$ or relational expressions. The above described processing up to the storage of the constants $\alpha$, $\beta$, $\gamma$, $\alpha'$, $\beta'$, and $\gamma'$ or relational expressions in the relation information storage unit 371 is executed before transmission of ultrasound to an observation target, desirably upon factory shipment. Thereafter, transmission of ultrasound to the observation target is executed.

When the input unit 35 receives input of an instruction for instructing scanning of an observation target, transmission of ultrasound to the observation target is started, and the transmitting and receiving unit 31 receives an echo signal from the ultrasound transducer 21. Thereafter, the transmitting and receiving unit 31, the frequency analysis unit 331, and the single regression analysis unit 332 execute processing similar to that in the first embodiment, on the echo signal from the observation target. Based on the ultrasound echo from the observation target, the single regression analysis unit 332 thus calculates a feature c and a feature a of the observation target, for which attenuation has been corrected appropriately by the attenuation correction unit 332b, and outputs them to the estimating unit 333. Processing by the estimating unit 333 and the physical quantity image data generating unit 342 is similar to that in the above described first embodiment.

According to the above described third embodiment, the above described effects of the first embodiment are able to be obtained, and since the constants $\alpha$, $\beta$, $\gamma$, $\alpha'$, $\beta'$, and $\gamma'$ are found in the ultrasound diagnosis device 3, in a case where an ultrasound endoscope having a new ultrasound transducer has been added, or a reference phantom has been added, update is able to be executed device by device.

Further, according to the third embodiment of the present invention, the constants $\alpha$, $\beta$, $\gamma$, $\alpha'$, $\beta'$, and $\gamma'$ are found in the ultrasound diagnosis device 3. Therefore, even if a property, such as transmission and reception sensitivity, differs among ultrasound transducers, calculation of the constants $\alpha$, $\beta$, $\gamma$, $\alpha'$, $\beta'$, and $\gamma'$ per body of ultrasound transducers is facilitated, and correspondingly to each ultrasound transducer, regardless of variation thereof, the number density n of scattering bodies, its logarithm $\log n$, the diameter d of scattering bodies, and its logarithm $\log d$ are able to be estimated more accurately and easily.

Further, even if properties of the ultrasound transducer change over time, periodic calculation of the constants $\alpha$, $\beta$, $\gamma$, $\alpha'$, $\beta'$, and $\gamma'$ is facilitated, and regardless of the change over time, the estimation is able to be executed more accurately.

Further, according to the third embodiment of the present invention, since the constants $\alpha$, $\beta$, $\gamma$, $\alpha'$, $\beta'$, and $\gamma'$ are found based on the features that are from the reference phantoms and that have been subjected to attenuation correction, $\log n$ and $\log d$ are able to be estimated more accurately, independently of the differences between the attenuation factors of the observation target and the reference phantoms.

Further, according to the third embodiment of the present invention, even if the number density $n_i$ of scattering bodies of a reference phantom or the diameter $d_i$ of the scattering bodies does not directly have a linear relation to the feature $c_i$ or $a_i$, log $n_i$ or log $d_i$ that has a substantially linear relation to the feature $c_i$ or $a_i$ is able to be used. Imaging of information on the number density n of scattering bodies and the diameter d of scattering bodies of the observation target, which are the truly desired physical quantities, are able to be realized by reduction to linear multiple regression analysis.

The variable transformation unit 38 and the multiple regression analysis unit 39 according to the third embodiment may be applied to the above described modified example of the first embodiment or second embodiment.

Fourth Embodiment

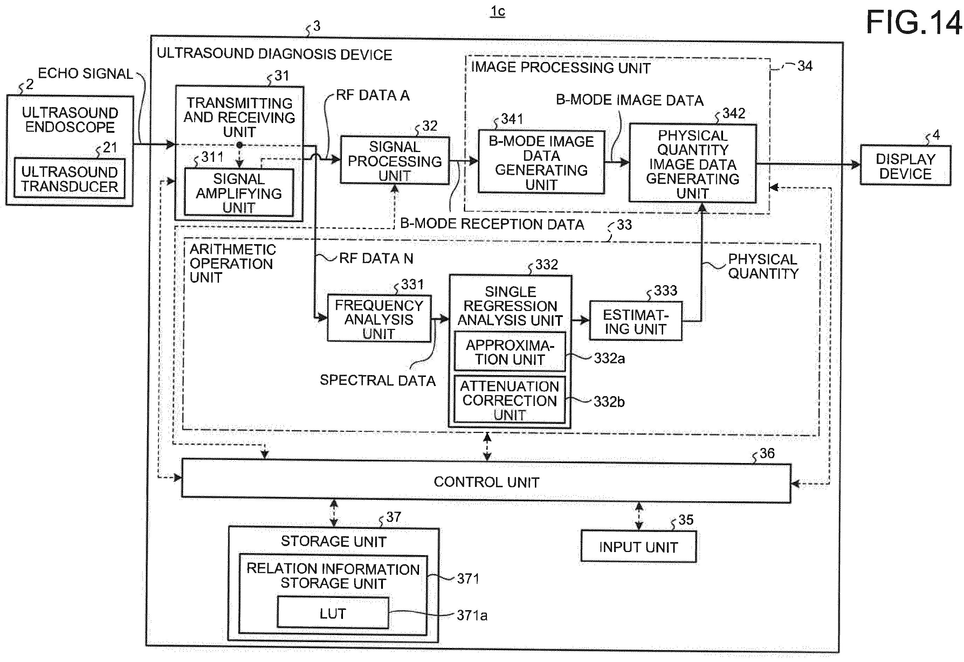

Next, a fourth embodiment of the present invention will be described. According to the above description of the first embodiment, the relation information storage unit 371 stored therein the constants $\alpha$, $\beta$, $\gamma$, $\alpha'$, $\beta'$, and $\gamma'$ or relational expressions beforehand, but in this fourth embodiment, the relation information storage unit 371 stores therein a lookup table (LUT) that is able to output a physical quantity according to a feature value. FIG. 14 is a block diagram illustrating a configuration of an ultrasound diagnosis system including an ultrasound diagnosis device according to the fourth embodiment of the present invention.

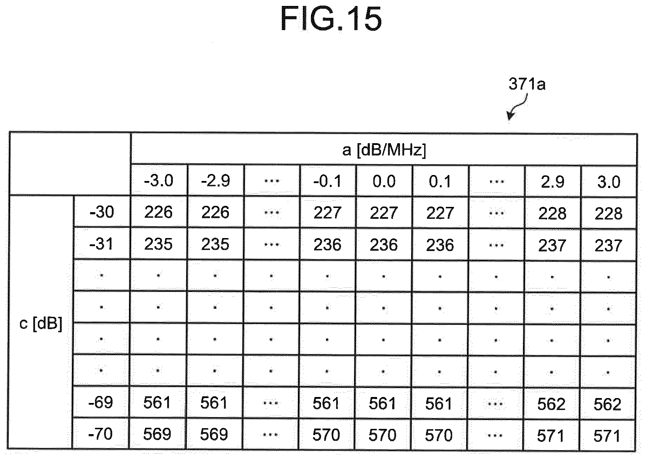

In an ultrasound diagnosis system 1c according to this fourth embodiment, in contrast to the above described configuration of the ultrasound diagnosis system 1 according to the first embodiment, the relation information storage unit 371 stores therein an LUT 371a. FIG. 15 is a diagram for explanation of a lookup table stored in the ultrasound diagnosis device according to the fourth embodiment of the present invention. FIG. 15 illustrates, as an example, a lookup table for output of, as a physical quantity, log n that is a logarithm of the number density n of scattering bodies, by input of features a and c.

Where the LUT 371a is a lookup table for output of log n, for example, the LUT 371a is generated by: calculation of each of log n and log d by Equation (10) above based on the constants $\alpha$, $\beta$, $\gamma$, $\alpha'$, $\beta'$, and $\gamma'$ obtained by the above described multiple regression analysis and plural provisional features a and c extracted at predetermined intervals; and substitution of a value of log n into each cell with the vertical axis being the provisional feature c and the horizontal axis being the provisional feature a. For log d, similarly, based on the constants $\alpha$, $\beta$, $\gamma$, $\alpha'$, $\beta'$, and $\gamma'$ and the provisional features a and c, a lookup table for output of log n is able to be generated. The LUT 371a may be generated in the ultrasound diagnosis device 3; the LUT 371a generated by an arithmetic operation device outside the ultrasound diagnosis device 3 may be obtained; or the LUT 371a may be obtained via a network. When the lookup table is generated, a feature $c_i$, a feature $a_i$, and an attenuation factor $\zeta_i$ of N reference phantoms are used ($1 \leq i \leq N$). The look-up table is generated after influence of attenuation is eliminated from these features by use of: the attenuation factors; the depths, at which the spectral data of the reference phantoms have been calculated; Equation (5); and Equation (3), such that the features are not dependent on the depth.

The relation information storage unit 371 stores therein, as LUTs 371a, a lookup table for output of log n, and a look-up table for output of log d. When the estimating unit 333 receives the features a and c from the single regression analysis unit 332, the estimating unit 333 rounds, for example, the first decimal place of the feature a and the second decimal place of the feature c. Thereafter, when outputting log n, the estimating unit 333 reads and refers to the lookup table for output of log n from the relation information storage unit 371, and estimates log n. Further, when outputting log d, the estimating unit 333 reads and refers to the lookup table for output of log d from the relation information storage unit 371, and estimates log d.

According to the above described fourth embodiment of the present invention, the above described effects of the first embodiment are able to be obtained, and the processing time needed for calculation of physical quantities is able to be shortened.

Further, according to the fourth embodiment of the present invention, when a lookup table is generated, the lookup table is generated after influence of attenuation has been eliminated from features of reference phantoms and the features are made to be not dependent on depth; and thus there is no need for lookup tables to be prepared for different depths, the amount of data is small, and the processing is simple.

Further, in the above described modified example of the first embodiment, or second embodiment, the LUT 371a according to this fourth embodiment may be stored.

Thus far, modes for implementation of the present invention have been described, but the present invention is not to be limited only to the above described embodiments. For example, an ultrasound diagnosis device may be configured by connection of circuits having the functions via a bus, or may be configured such that some of the functions are built in a circuit structure of the other functions.

Further, according to the above description of the first to fourth embodiments, the size of scattering bodies is the diameter, but the size may be the radius or volume.

Further, according to the above description of the first to fourth embodiments, the example of the reference object is a reference phantom having scattering bodies mixed uniformly in a medium, for which the material, mass density, sound velocity, and acoustic impedance are known; the material, mass density, sound velocity, acoustic impedance, diameter, and number density of the scattering bodies also being known. However, any target, which has scattering bodies with known physical quantities, such as the diameter of the scattering bodies, the scattering intensity of the scattering bodies, and the number density of the scattering bodies, and in which the scattering bodies are uniformly distributed, may be used instead as the reference phantom. For example, as long as the physical quantities are known or are able to be measured accurately, a particular tissue, such as that of the liver of an animal, may be used.

Further, in the above description of the first to fourth embodiments, the number density n of scattering bodies, the diameter d of scattering bodies, and the scattering intensity r of scattering bodies are given as an example of the physical quantities. Furthermore, the slope a of the spectrum, the intercept b of the spectrum, the mid-band fit c of the spectrum, and the attenuation factor $\zeta$ are given as an example of the features. However, the physical quantities or the features may be a part of these or all of these. Further, the physical quantities or the features may be other physical quantities or features. For example, a quantity representing a shape of scattering bodies, or, for example, a fractal dimension representing atypism may be used. Moreover, as a physical quantity, for example, a dispersion of a distribution of diameters of scattering bodies may be used. A sound velocity or a mass density may be used as a feature.

Further, in the above described first to fourth embodiments, when the number of physical quantities estimated by the estimating unit 333 is plural, the plural physical quantities may be displayed concurrently on the display device 4, may be sequentially displayed in turn according to input of instructions through the input unit 35, or may be displayed at different timings (frames).

Further, according to the above description of the first to fourth embodiments, the ultrasound endoscope 2 having an optical system, such as a light guide, is used as the ultrasound probe, but the ultrasound probe is not limited to the ultrasound endoscope 2, and may be an ultrasound probe not having an imaging optical system and an imaging element. Furthermore, an ultrasound miniature probe without an optical system and having a small diameter may be applied as the ultrasound probe. The ultrasound miniature probe is normally inserted in a biliary tract, a bile duct, a pancreatic duct, a trachea, a bronchus, a urethra, or a ureter, and is used for observation of organ/organs therearound (a pancreas, lungs, a prostate, a bladder, a lymph node, and/or the like).

Further, an external ultrasound probe that emits ultrasound from a body surface of an observation target may be applied as the ultrasound probe. The external ultrasound probe is normally used by directly being contacted with the body surface when an abdominal organ (a liver, gallbladder, or bladder), breasts (mammary glands, in particular), or a thyroid gland is/are observed.

Further, the ultrasound transducer may be a linear transducer, a radial transducer, or a convex transducer. If the ultrasound transducer is a linear transducer, its scanning region is rectangular (rectangular or square), and if the ultrasound transducer is a radial transducer or a convex transducer, its scanning region is fan-shaped or toric. Further, in the ultrasound endoscope, the ultrasound transducer may be made to perform scanning mechanically; or plural elements may be provided in an array as the ultrasound transducer, and may be made to perform scanning electronically by electronic change of elements related to transmission and reception or insertion of delay in transmission and reception by the elements.

As described above, the present invention may include various embodiments without departing from the technical ideas stated in the claims.

Industrial Applicability

As described hereinbefore, an ultrasound diagnostic device, an operation method of the ultrasound diagnosis device, and an operation program for the ultrasound diagnosis device, according to the present invention, are useful for easy and accurate identification of tissue characteristics based on features. Reference Signs List

[0149]

　　1, 1a, 1b, 1c ULTRASOUND DIAGNOSIS SYSTEM
　　2 ULTRASOUND ENDOSCOPE
　　3 ULTRASOUND DIAGNOSIS DEVICE
　　4 DISPLAY DEVICE
　　21 ULTRASOUND TRANSDUCER
　　31 TRANSMITTING AND RECEIVING UNIT
　　32 SIGNAL PROCESSING UNIT
　　33 ARITHMETIC OPERATION UNIT
　　34 IMAGE PROCESSING UNIT
　　35 INPUT UNIT
　　36 CONTROL UNIT

37 STORAGE UNIT
38, 334 VARIABLE TRANSFORMATION UNIT
39 MULTIPLE REGRESSION ANALYSIS UNIT
201 FEATURE IMAGE
202 SUPERIMPOSED IMAGE DISPLAY SECTION
203 INFORMATION DISPLAY SECTION
331 FREQUENCY ANALYSIS UNIT
332 SINGLE REGRESSION ANALYSIS UNIT
332a APPROXIMATION UNIT
332b ATTENUATION CORRECTION UNIT
333 ESTIMATING UNIT
341 B-MODE IMAGE DATA GENERATING UNIT
342 PHYSICAL QUANTITY IMAGE DATA GENERATING UNIT
371 RELATION INFORMATION STORAGE UNIT

**Claims**

1. An ultrasound diagnosis device configured to generate an ultrasound image based on an ultrasound signal obtained by an ultrasound probe including an ultrasound transducer that transmits ultrasound to an observation target and receives ultrasound back-scattered by the observation target, the ultrasound diagnosis device comprising:

   a feature calculating unit configured to calculate a feature based on an ultrasound signal received from the observation target;
   an estimating unit configured to estimate a physical quantity of scattering bodies included in the observation target, by using: a relation derived based on a known physical quantity of a reference object and a feature obtained from the target object, the reference object including scattering bodies having the known physical quantity; and the feature of the observation target calculated by the feature calculating unit; and
   a physical quantity information generating unit configured to generate information to be displayed on a display unit, the information including a result of the estimation by the estimating unit.

2. The ultrasound diagnosis device according to claim 1, wherein the physical quantity includes at least one of a number density of the scattering bodies included in the reference object, a size of the scattering bodies, and a scattering intensity of the scattering bodies.

3. The ultrasound diagnosis device according to claim 1, wherein
   the physical quantity includes a scattering intensity of the scattering bodies included in the reference object, and
   the scattering intensity is at least one of an amplitude reflectance of the scattering bodies and a medium, an energy reflectance of the scattering bodies and a medium, and a function of the scattering bodies and a medium.

4. The ultrasound diagnosis device according to claim 1, wherein the estimating unit is configured to estimate the physical quantity of the scattering bodies included in the observation target by substituting the feature calculated by the feature calculating unit into a relational expression serving as the relation.

5. The ultrasound diagnosis device according to claim 4, further comprising:

   a relation information storage unit configured to store therein at least one of: the relational expression; a coefficient of the relational expression; a constant term of the relational expression; and a table describing therein the relation, wherein
   the estimating unit is configured to estimate the physical quantity of the scattering bodies included in the observation target, by referring to the relation information storage unit.

6. The ultrasound diagnosis device according to claim 1, wherein the relational expression is derived by multiple regression analysis of at least a part of the physical quantity and the feature.

7. The ultrasound diagnosis device according to claim 6, wherein if the known physical quantity of the scattering bodies includes a number density of the scattering bodies and a size of the scattering bodies, the multiple regression analysis is executed by non-linear transformation of the number density of the scattering bodies and the scattering bodies.

8. The ultrasound diagnosis device according to claim 1, wherein the feature includes a frequency feature calculated based on the ultrasound signal.

9. The ultrasound diagnosis device according to claim 1, wherein the feature includes an attenuation factor calculated based on the ultrasound signal.

10. The ultrasound diagnosis device according to claim 1, wherein the feature includes a sound velocity calculated based on the ultrasound signal.

11. The ultrasound diagnosis device according to claim 1, wherein the physical quantity information generating unit is configured to generate image data added with visual information according to the physical quantity estimated by the estimating unit.

12. The ultrasound diagnosis device according to claim 1, wherein if the estimating unit estimates plural physical quantities different from one another, the physical quantity information generating unit is configured to generate the information displayed on the display unit concurrently, sequentially, or at different timings, for the plural physical quantities.

13. The ultrasound diagnosis device according to claim 6, further comprising a variable transformation unit configured to execute, on at least one of the number density of the scattering bodies that has been subjected to the non-linear transformation and the size of the scattering bodies that has been subjected to the non-linear transformation, further non-linear transformation.

14. The ultrasound diagnosis device according to claim 1, further comprising:

an attenuation correction unit configured to correct influence of attenuation in the feature calculated based on an ultrasound signal received from the observation target, wherein
the estimating unit is configured to estimate the physical quantity of the scattering bodies included in the observation target by using: a relation derived based on the known physical quantity of the reference object including the scattering bodies having the known physical quantity and a value resulting from correction of influence of attenuation in the feature obtained from the reference object; and a value of the feature of the observation target corrected by the attenuation correction unit.

15. An operation method of an ultrasound diagnosis device configured to generate an ultrasound image based on an ultrasound signal obtained by an ultrasound probe including an ultrasound transducer that transmits ultrasound to an observation target and receives ultrasound back-scattered by the observation target, the operation method including:

a feature calculating step of a feature calculating unit calculating a feature based on an ultrasound signal received from the observation target;
an estimating step of an estimating unit estimating a physical quantity of scattering bodies included in the observation target, by using: a relation derived based on a known physical quantity of a reference object and a feature obtained from the target object, the reference object including scattering bodies having the known physical quantity; and the feature of the observation target calculated by the feature calculating unit; and
a physical quantity information generating step of a physical quantity information generating unit generating information to be displayed on a display unit, the information including a result of the estimation by the estimating unit.

16. An operation program for an ultrasound diagnosis device configured to generate an ultrasound image based on an ultrasound signal obtained by an ultrasound probe including an ultrasound transducer that transmits ultrasound to an observation target and receives ultrasound back-scattered by the observation target, the operation program causing the ultrasound diagnosis device to execute:

feature calculating of a feature calculating unit calculating a feature based on an ultrasound signal received from the observation target;
estimating of an estimating unit estimating a physical quantity of scattering bodies included in the observation target, by using: a relation derived based on a known physical quantity of a reference object and a feature obtained from the target object, the reference object including scattering bodies having the known physical quantity; and the feature of the observation target calculated by the feature calculating unit; and

physical quantity information generating of a physical quantity information generating unit generating information to be displayed on a display unit, the information including a result of the estimation by the estimating unit.

# FIG.1

ECHO SIGNAL

ULTRASOUND ENDOSCOPE 2
ULTRASOUND TRANSDUCER 21

ULTRASOUND DIAGNOSIS DEVICE 3

TRANSMITTING AND RECEIVING UNIT 31
SIGNAL AMPLIFYING UNIT 311

RF DATA A
SIGNAL PROCESSING UNIT 32

B-MODE RECEPTION DATA

RF DATA N
ARITHMETIC OPERATION UNIT 33
FREQUENCY ANALYSIS UNIT 331

SPECTRAL DATA

SINGLE REGRESSION ANALYSIS UNIT 332
APPROXIMATION UNIT 332a
ATTENUATION CORRECTION UNIT 332b

ESTIMATING UNIT 333

PHYSICAL QUANTITY

IMAGE PROCESSING UNIT 34
B-MODE IMAGE DATA GENERATING UNIT 341
B-MODE IMAGE DATA
PHYSICAL QUANTITY IMAGE DATA GENERATING UNIT 342

DISPLAY DEVICE 4

CONTROL UNIT 36

INPUT UNIT 35

STORAGE UNIT 37
RELATION INFORMATION STORAGE UNIT 371

1

25

# FIG.2

# FIG.3

# FIG.4

EP 3 387 998 A1

# FIG.5

# FIG.6

# FIG.7

# FIG.8

```
                    ┌─────────┐
                    │  START  │
                    └────┬────┘
                         │
         ┌──────────────▶▼────────────────────┐
         │  RECEIVE RESULT OF MEASUREMENT     │ ∿S1
         │      ON OBSERVATION TARGET         │
         └────────────────┬───────────────────┘
                          │
         ┌────────────────▼───────────────────┐
         │   AMPLIFY RECEIVED ECHO SIGNAL     │ ∿S2
         │         (STC CORRECTION)           │
         └────────────────┬───────────────────┘
                          │
         ┌────────────────▼───────────────────┐
         │     GENERATE B-MODE IMAGE DATA     │ ∿S3
         └────────────────┬───────────────────┘
                          │
         ┌────────────────▼───────────────────┐
         │       DISPLAY B-MODE IMAGE         │ ∿S4
         └────────────────┬───────────────────┘
                          │
                          ▼                     ⌐S5
    NO        ◇ DISPLAY PHYSICAL ◇
 ◀────────────◇ QUANTITY IMAGE?  ◇
              ◇                  ◇
                          │ YES
                          ▼
         ┌────────────────▼───────────────────┐
         │        FREQUENCY ANALYSIS          │ ∿ S6
         └────────────────┬───────────────────┘
                          │
         ┌────────────────▼───────────────────┐
         │      CALCULATE UNCORRECTED         │ ∿S7
         │           FEATURES                 │
         └────────────────┬───────────────────┘
                          │
         ┌────────────────▼───────────────────┐
         │  EXECUTE ATTENUATION CORRECTION    │ ∿S8
         │     ON UNCORRECTED FEATURES        │
         └────────────────┬───────────────────┘
                          │
         ┌────────────────▼───────────────────┐
         │     ESTIMATE logn AND/OR logd      │ ∿S9
         └────────────────┬───────────────────┘
                          │
         ┌────────────────▼───────────────────┐
         │    GENERATE PHYSICAL QUANTITY      │ ∿S10
         │           IMAGE DATA               │
         └────────────────┬───────────────────┘
                          │
         ┌────────────────▼───────────────────┐
         │  DISPLAY PHYSICAL QUANTITY IMAGE   │ ∿S11
         └────────────────┬───────────────────┘
                          │
                    ┌─────▼────┐
                    │   END    │
                    └──────────┘
```

# FIG.9

```
        ┌──────────────┐
        │  FREQUENCY   │
        │  ANALYSIS    │
        └──────┬───────┘
               ▼
        ┌──────────────┐
        │   k=k₀       │─── S21
        └──────┬───────┘
               ▼
        ┌──────────────┐
        │  Z⁽ᵏ⁾=Z⁽ᵏ⁾₀  │─── S22
        └──────┬───────┘
```

$k = k_0$ — S21

$Z^{(k)} = Z^{(k)}_0$ — S22

ACQUIRE SAMPLE DATA GROUP — S23

APPLY WINDOW FUNCTION — S24

S25
SAMPLE DATA GROUP IS NORMAL?

NO

S26
INSERT ZERO DATA WORTH DEFICIENCY

YES

FFT ARITHMETIC OPERATIONS — S27

$Z^{(k)} = Z^{(k)} + D$ — S28

S29
$Z^{(k)} > Z^{(k)}_{max}$?

NO

YES

$k = k + 1$ — S30

S31
$k > k_{max}$?

NO

YES

RETURN

# FIG.10

FIG.11

EP 3 387 998 A1

# FIG.12

ID: XXXXX

NUMBER
DENSITY: YYY

FIG.13

ULTRASOUND DIAGNOSIS DEVICE

IMAGE PROCESSING UNIT

ECHO SIGNAL

ULTRASOUND ENDOSCOPE

ULTRASOUND TRANSDUCER

TRANSMITTING AND RECEIVING UNIT

SIGNAL AMPLIFYING UNIT

RF DATA A

SIGNAL PROCESSING UNIT

B-MODE IMAGE DATA

B-MODE IMAGE DATA GENERATING UNIT

PHYSICAL QUANTITY IMAGE DATA GENERATING UNIT

DISPLAY DEVICE

B-MODE RECEPTION DATA

ARITHMETIC OPERATION UNIT

RF DATA N

FREQUENCY ANALYSIS UNIT

SPECTRAL DATA

SINGLE REGRESSION ANALYSIS UNIT

APPROXIMA-TION UNIT

ATTENUATION CORRECTION UNIT

ESTIMAT-ING UNIT

PHYSICAL QUANTITY

CONTROL UNIT

VARIABLE TRANSFORMA-TION UNIT

MULTIPLE REGRESSION ANALYSIS UNIT

STORAGE UNIT

RELATION INFORMATION STORAGE UNIT

INPUT UNIT

EP 3 387 998 A1

FIG.14

EP 3 387 998 A1

# FIG.15

371a

| | | a [dB/MHz] | | | | | | | | |
|---|---|---|---|---|---|---|---|---|---|---|
| | | -3.0 | -2.9 | ··· | -0.1 | 0.0 | 0.1 | ··· | 2.9 | 3.0 |
| c [dB] | -30 | 226 | 226 | ··· | 227 | 227 | 227 | ··· | 228 | 228 |
| | -31 | 235 | 235 | ··· | 236 | 236 | 236 | ··· | 237 | 237 |
| | . | . | . | . | . | . | . | . | . | . |
| | . | . | . | . | . | . | . | . | . | . |
| | . | . | . | . | . | . | . | . | . | . |
| | . | . | . | . | . | . | . | . | . | . |
| | -69 | 561 | 561 | ··· | 561 | 561 | 561 | ··· | 562 | 562 |
| | -70 | 569 | 569 | ··· | 570 | 570 | 570 | ··· | 571 | 571 |

## INTERNATIONAL SEARCH REPORT

| International application No. |
|---|
| PCT/JP2016/084797 |

| A. CLASSIFICATION OF SUBJECT MATTER |
|---|
| *A61B8/12*(2006.01)i |

According to International Patent Classification (IPC) or to both national classification and IPC

| B. FIELDS SEARCHED |
|---|

Minimum documentation searched (classification system followed by classification symbols)
A61B8/12

Documentation searched other than minimum documentation to the extent that such documents are included in the fields searched

| Jitsuyo Shinan Koho | 1922–1996 | Jitsuyo Shinan Toroku Koho | 1996–2016 |
| Kokai Jitsuyo Shinan Koho | 1971–2016 | Toroku Jitsuyo Shinan Koho | 1994–2016 |

Electronic data base consulted during the international search (name of data base and, where practicable, search terms used)

| C. DOCUMENTS CONSIDERED TO BE RELEVANT |
|---|

| Category* | Citation of document, with indication, where appropriate, of the relevant passages | Relevant to claim No. |
|---|---|---|
| A | JP 5659324 B1  (Olympus Medical Systems Corp.),<br>28 January 2015 (28.01.2015),<br>paragraphs [0028] to [0042]<br>& US 2015/0178919 A1<br>paragraphs [0039] to [0057]<br>& WO 2014/192954 A1    & EP 3005945 A1<br>& CN 104582584 A | 1–16 |
| A | JP 2013-166059 A  (Olympus Medical Systems Corp.),<br>29 August 2013 (29.08.2013),<br>paragraphs [0026] to [0090]<br>& US 2013/0035594 A1<br>paragraphs [0033] to [0099]<br>& WO 2012/063928 A1    & EP 2599440 A1<br>& CN 103200876 A | 1–16 |

[X] Further documents are listed in the continuation of Box C.          [ ] See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 12 December 2016 (12.12.16) | 20 December 2016 (20.12.16) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

International application No.

PCT/JP2016/084797

C (Continuation).   DOCUMENTS CONSIDERED TO BE RELEVANT

| Category* | Citation of document, with indication, where appropriate, of the relevant passages | Relevant to claim No. |
|---|---|---|
| A | WO 2013/179859 A1  (Olympus Medical Systems Corp.),<br>05 December 2013 (05.12.2013),<br>entire text; all drawings<br>& JP 5430809 B1        & US 2014/0099008 A1<br>ntire text; all drawings<br>& EP 2719337 A1          & CN 103717146 A | 1-16 |
| A | WO 2012/063976 A1  (Olympus Medical Systems Corp.),<br>18 May 2012 (18.05.2012),<br>entire text; all drawings<br>& JP 5054254 B2        & US 2012/0310087 A1<br>entire text; all drawings<br>& EP 2548512 A1          & CN 102802536 A | 1-16 |

Form PCT/ISA/210 (continuation of second sheet) (January 2015)

## REFERENCES CITED IN THE DESCRIPTION

**Patent documents cited in the description**

- WO 2012011414 A